# EUROPEAN PATENT APPLICATION

(11) **EP 1 977 644 A1**
(43) Date of publication of application: **08.10.2008**
(21) Application number: 07006907.5
(22) Date of filing: 03.04.2007
(51) Int. Cl.: A01N 43/76, C07D 263/28, A01P 5/00, A01P 7/00, A01P 9/00

(54) **Insecticidal derivatives of substituted benzylamines**

(71) Applicant: Bayer CropScience Aktiengesellschaft, 40789 Monheim (DE)
(72) Inventor: Andree, Roland, Dr., 40764 Langenfeld (DE); Schwarz, Hans-Georg, Dr., 40764 Langenfeld (DE); Schallner, Otto, Dr., 40789 Monheim (DE); Holmwood, Graham, Dr., 51371 Leverkusen (DE)

(57) **Abstract**

The present invention relates to novel derivatives of substituted benzylamines, to processes for their preparation and to their use for controlling animal pests, especially arthropods, in particular insects.

## Description

The present application relates to novel insecticidal derivatives of substituted benzylamines, to processes for their preparation and to their use for controlling animal pests, especially arthropods, in particular insects.

Insecticidal and acaricidal benzylamino heterocyclic derivatives of the general formula 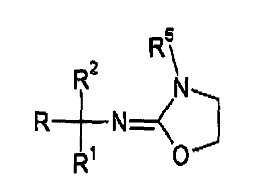 wherein
R is 1-naphthyl, phenyl or phenyl substituted with one or two substituents selected from halogen or (C₁-C₂) alkyl;
R¹ is selected from hydrogen, (C₁-C₄) alkyl and (C₁-C₂) haloalkyl; R² is hydrogen; R⁵ is selected from cyano, (C₁-C₂) alkoxy(C₁-C₂) alkyl, 4-(C₁-C₂) alkoxybenzyl, 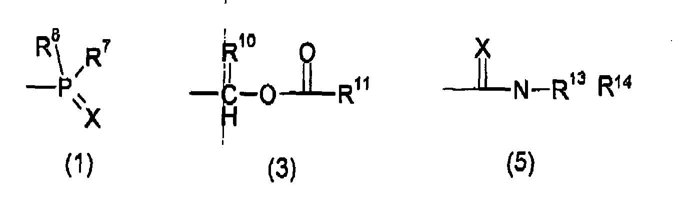 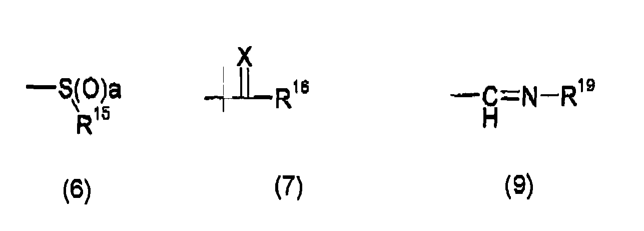 where
X is oxygen or sulfur; R⁷ and R⁸ are (C₁-C₂) alkoxy or (C₁-C₂) haloalkyl; R¹⁰ is hydrogen; R¹¹ is (C₁-C₄) alkyl; R¹³ is (C₁-C₂) alkyl; R¹⁴ is hydrogen or (C₁-C₂) alkyl; a is 2; R¹⁵ is (C₁-C₂) alkyl or (C₁-C₂) dialkylamino; R¹⁶ is (C₁-C₂) alkyl or (C₁-C₂) alkoxy; and R¹⁹ is (C₁-C₂) alkyl or (C₁-C₂) alkoxy; provided that when R is 1-naphthyl and R⁵ is formula (5) where X is sulfur, R¹³ is methyl and R¹⁴ is hydrogen, then R¹ is other than (C₁-C₂) alkyl; and when R is 3-chloro-2-methylphenyl and R¹ is hydrogen, then R⁵ is other than formula (5) where X is oxygen, R¹³ is methyl and R¹⁴ is hydrogen or formula (6) where a is 2 and R¹⁵ is methyl,
are described as one embodiment in WO 2006/127426 (cf. e.g, formula IB, page 8).

There is however a continuing demand for new insecticides and acaricides that are not only safer and less costly than the known compounds but are in particular more effective.

This invention now provides novel compounds of the formula (I) 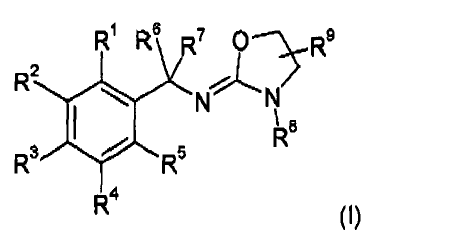 in which
R¹, R², R³, R⁴, and R⁵ independently from each other represent hydrogen, halogen, hydroxy, alkyl, alkoxy, haloalkyl, alkoxyalkyl, cycloalkyl, cyanoalkyl, haloalkoxy, alkylthio, haloalkylthio, alkylsulfonyl, alkylsulfonyloxy, halogenalkylsulfonyl, halogenalkylsulfonyloxy, alkoxycarbonyl, acetyl, alkylcarbonyl, alkenylcarbonyl, pentafluorosulfanyl, amino, mono- and dialkylamino, cycloalkylamino, alkenyl, haloalkenyl, alkynyl, haloalkynyl, cyano, or nitro, or represent independently from each other aryl, aryloxy or heteroaryl which are optionally substituted with one or more substituents selected from halogen, alkyl, haloalkyl, alkoxy, haloalkoxy, alkoxycarbonyl, nitro, and cyano;
R⁶ and R⁷ independently from each other represent hydrogen, alkyl, haloalkyl, cycloalkyl, alkoxyalkyl, alkylmercaptoalkyl, alkenyl, or alkynyl, or represent independently from each other aryl, heteroaryl or heterocyclyl, optionally substituted with one or more substituents selected from halogen, alkyl, alkoxy, nitro, and cyano;
R⁸ represents -C(Z)R¹⁰, -C(Z)OR¹⁰, or C(Z)NR¹¹R¹²;
Z represents O or S;
R⁹ represents hydrogen, alkyl, or haloalkyl;
R¹⁰ represents C₁-C₂ alkyl, substituted with one or more substituents selected from halogen, alkoxy, alkylmercapto and cyano, or represents C₃-C₆ alkyl, cycloalkyl, or benzyl, optionally substituted with one or more substituents selected from halogen, and alkoxy;
R¹¹ represents C₁-C₂ alkyl, substituted with one or more substituents selected from halogen, alkoxy, alkylmercapto, alkoxycarbonyl, alkylsulfinyl, alkylsulfonyl and cyano, or represents C₃-C₈ alkyl, cycloalkyl, cycloalkylalkyl, alkenyl, or alkynyl, optionally substituted with one or more substituents selected from halogen, haloalkyl, alkoxy, alkylmercapto, alkylsulfinyl, alkylsulfonyl, dialkylamino, alkoxycarbonyl, and dialkylaminocarbonyl, or represents arylalkyl, heterocyclylalkyl aryl, heteroaryl, or heteroarylalkyl, optionally substituted with one or more substituents selected from halogen, alkyl, halogenalkyl, alkoxy, halogenalkoxy, alkoxycarbonyl, heterocyclyl, dialkylammocarbonyl, cyano, nitro, dialkylamino, S(O)ₙ-alkyl, and S(O)ₙ-halogenalkyl;
R¹² represents hydrogen or alkyl, cycloalkyl, alkenyl, alkynyl, optionally substituted with one or more substituents selected from halogen, alkoxy, alkylmercapto, cyano, alkylsulfinyl, alkylsulfonyl, dialkylamino, alkoxycarbonyl, and dialkylaminocarbonyl, or represents arylalkyl, heterocyclylalkyl, aryl, or heteroaryl, optionally substituted with one or more substituents selected from halogen, alkyl, halogenalkyl alkoxy, halogenalkoxy, alkoxycarbonyl, dialkylaminocarbonyl, cyano, nitro, dialkylamino, S(O)ₙ-alkyl, and S(O)ₙ-halogenalkyl; and
nis 0, 1 or 2.

Depending inter alia on the nature of the substituents, the compounds of the present invention may be present as geometrical and/or as optically active isomers or corresponding isomer mixtures of varying composition. Molecules that are not superimposable on their mirror image are called chiral; these molecules are optically active. If a molecule is nonsuperimposible on its mirror image, the mirror image must be a different molecule, since superimposability is the same as identity, In each case of optical activity of a pure compound there are two and only two isomers, called enantiomers, which differ in structure only in the left- and right-handedrness of their orientations. Enantiomers differ in that they rotate the plane of polarized light in opposite directions and in that they react at different rates with other chiral compounds or in the presence of a chiral catalyst (cf, March's Advanced Organic Chemistry 5th edition; Wiley 2001 page 125-126).

Provided that substituents R⁶ and R⁷ are different, the C-atom they are attached to is asymmetric or chiral and the respective compound is optically active. According to the Cahn-Ingold-Prelog system that ranks the four groups on an asymmetric carbon atom in order of decreasing atomic number, the asymmetric C-atom is present in S- or R-confgurataon (cf. March's Advanced Organic Chemistry 5th edition ; Wiley 2001 page 139).

Compounds in which rotation is restricted may exhibit cis-trans isomerism. One type of cis-trans isomerism results from double bonds, such as the N=Oxazolidme-double bond. Based on the Cahn-Ingold-Prelog system, ranking the groups at each atom of the double bond in order of decreasing atomic number, the compounds according to the invention can be present in the Z or E form, Z being the isomer with the two higher ranking groups on the same side of the double bond (cf. March's Advanced Organic Chemistry 5th edition, Wiley 2001 page 157-158). 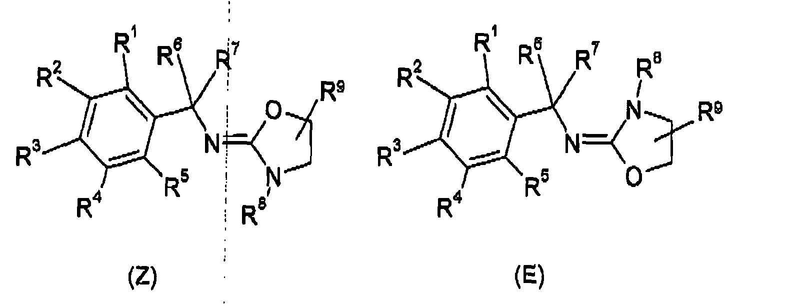 Formula (I) of the present invention comprises both the pure R and S enantiomers and the enantiomer mixtures or racemates. Preferred are compounds of formula (Ib) 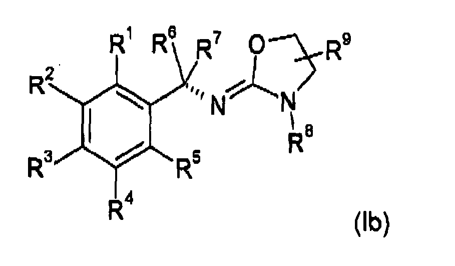 in which R¹ to R⁹ are as defined above for formula (I). Depending on the actual substituents R¹ to R⁷, the asymmetric C-atom might be present in the R- or the S-configuration.

There is no disclosure or suggestion in WO 2006/127426 of the structures of the novel compounds of formula (I) of the present invention, In particular, there is no disclosure or suggestion in in WO 2006/127426 of the surprisingly and significantly improved insecticidal activity of the compounds of formula (Ib) over their optical antipodes or the racemates, respectively.

As used the "optical antipode" of an S-enantiomer is defined as the corresponding R-enantiomer that differs only in the configuration of the asymmetric C-atom and rotates the plane of polarized light in the opposite direction. Accordingly, the optical antipode of an R-enantiomer is the corresponding S-enantiomer.

The formula (I) provides a general definition of the compounds according to the invention.

Preferred substituents or ranges of the radicals given in the formulae mentioned above and below are illustrated below.

According to a further preferred embodiment of the invention,
R¹, R², R³, R⁴, and R⁵ of formula (I) independently from each other represent hydrogen, halogen, preferably chlorine or fluorine, hydroxy, C₁-C₄ alkyl, preferably C₁-C₂ alkyl, C₁-C₄ alkoxy, preferably C₁-C₂ alkoxy, C₁-C₄ haloalkyl, preferably C₁-C₂ haloalkyl, (C₁-C₄)alkoxy(C₁-C₄)alkyl, preferably (C₁-C₂)alkoxy(C₁-C₂)alkyl, C₃-C₈ cycloalkyl, preferably C₃-C₆ cycloalkyl, cyano(C₁-C₄)alkyl, preferably cyano(C₁-C₂)alkyl, halo(C₁-C₄)alkoxy, preferably halo(C₁-C₂)alkoxy, C₁-C₄ alkylthio, preferably C₁-C₂ alkylthio, halo(C₁-C₄)alkylthio, preferably halo(C₁-C₂)alkylthio, C₁-C₄ alkylsulfonyl, preferably C₁-C₂ alkylsulfonyl, C₁-C₄ alkylsulfonyloxy, preferably C₁-C₂ alkylsulfonyloxy, halogen(C₁-C₄)alkylsulfonyl, preferably halogen(C₁-C₂)alkylsulfonyl, halogen(C₁-C₄)alkylsulfonyloxy, preferably halogen(C₁-C₂)alkylsulfonyloxy, C₁-C₄ alkoxycarbonyl, preferably C₁-C₂ alkoxycarbonyl, acetyl, C₁-C₄ alkylcarbonyl, preferably C₁-C₂ alkylcarbonyl, C₂-C₄ alkenylcarbonyl, pentafluorosulfanyl, amino, mono- and di(C₁-C₄)alkylamino, preferably mono- and di(C₁-C₂)alkylamino, C₃-C₈ cycloalkylamino, preferably C₃-C₆ cycloalkylamino, C₂-C₄ alkenyl, halo(C₂-C₄)alkenyl, C₂-C₄ alkynyl, halo(C₂-C₄)alkynyl, cyano, or nitro, or represent independently from each other C₅-C₈ aryl, preferably C₅-C₆ aryl, C₅-C₈ aryloxy, preferably C₅-C₆ aryloxy, or heteroaryl comprising a 5-8 membered ring containing one, two or more heteroatoms selected from 0, N, P, and S, which are optionally substituted with one or more substituents selected from halogen, C₁-C₄ alkyl, preferably C₁-C₂ alkyl, halo(C₁-C₄)alkyl, preferably halo(C₁-C₂)alkyl, C₁-C₄ alkoxy, preferably C₁-C₂ alkoxy, halo(C₁-C₄)alkoxy, preferably halo(C₁-C₂)alkoxy, C₁-C₄ alkoxycarbonyl, preferably C₁-C₂ alkoxycarbonyl, nitro, and cyano;
R⁶ and R⁷ independently from each other represent hydrogen, C₁-C₄ alkyl, preferably C₁-C₄ alkyl, C₁-C₄ haloalkyl, preferably C₁-C₂ haloalkyl, C₃-C₈ cycloalkyl, preferably C₃-C₆ cycloalkyl, (C₁-C₄)alkoxy(C₁-C₄)alkyl, preferably (C₁-C₂)alkoxy(C₁-C₂)alkyl, C₁-C₄ alkylmercapto(C₁-C₄)alkyl, preferably C₁-C₂ alkylmercapto(C₁-C₂)alkyl, C₂-C₄ alkenyl, or C₂-C₄ alkynyl, or represent independently from each other C₅-C₈ aryl, preferably C₅-C₆ aryl, heteroaryl comprising a 5-8 membered ring containing one, two or more heteroatoms selected from O, N, P, and S, or heterocyclyl comprising a 5-8 membered ring containing one, two or more heteroatoms selected from 0, N, P, and S, optionally substituted with one or more substituents selected from halogen, C₁-C₄ alkyl, preferably C₁-C₂ alkyl, C₁-C₄ alkoxy, preferably C₁-C₂ alkoxy, nitro, and cyano;
R⁸ represents -C(Z)R¹⁰, -C(Z)OR¹⁰, or -C(Z)NR¹¹R¹²;
Z represents O or S;
R⁹ represents hydrogen, C₁-C₄ alkyl, preferably C₁-C₂ alkyl, or C₁-C₄ haloalkyl, preferably C₁-C₂ haloalkyl;
R¹⁰ represents C₁-C₂ alkyl, substituted with one or more substituents selected from halogen, C₁-C₄ alkoxy, preferably C₁-C₂ alkoxy, C₁-C₄ alkylmercapto, preferably C₁-C₂ alkylmercapto, and cyano, or represents C₃-C₆ alkyl, C₃-C₆ cycloalkyl, or benzyl, optionally substituted with one or more sub-stituents selected from halogen, and C₁-C₄ alkoxy, preferably C₁-C₂ alkoxy;
R¹¹ represents C₁-C₂ alkyl, substituted with one or more substituents selected from halogen, C₁-C₄ alkoxy, preferably C₁-C₂ alkoxy, C₁-C₄ alkylmercapto, preferably C₁-C₂ alkylmercapto, C₁-C₄ alkoxycarbonyl, preferably C₁-C₂ alkoxycarbonyl, C₁-C₄ alkylsulfinyl, preferably C₁-C₂ alkylsulfinyl, C₁-C₄ alkylsulfonyl, preferably C₁-C₂ alkylsulfonyl, and cyano, or represents C₃-C₈ alkyl, C₃-C₆ cycloalkyl, (C₃-C₆)cycloalkyl(C₁-C₄)alkyl, C₂-C₄ alkenyl, or C₂-C₄ alkynyl, optionally substituted with one or more substituents selected from halogen, halo(C₁-C₄)alkyl, preferably halo(C₁-C₂)alkyl, C₁-C₄ alkoxy, preferably C₁-C₂ alkoxy, C₁-C₄ alkylmercapto, preferably C₁-C₂ alkylmercapto, C₁-C₄ alkylsulfinyl, preferably C₁-C₂ alkylsulfinyl, C₁-C₄ alkylsulfonyl, preferably C₁-C₂ alkylsulfonyl, di(C₁-C₄)alklamino, preferably di(C₁-C₂)alkylamino, C₁-C₄ alkoxycarbonyl, preferably C₁-C₂ alkoxycarbonyl, and di(C₁-C₄)alkylaminocarbonyl, preferably di(C₁-C₃)alkylaminocarbonyl, or represents C₅-C₈ aryl(C₁-C₄)alkyl, preferably C₅-C₆ aryl(C₁-C₂)alkyl, heterocyclyl(C₁-C₄)alkyl, preferably heterocyclyl(C₁-C₂)alkyl, the heterocyclyl comprising a 5-8 membered ring containing one, two or more heteroatoms selected from 0, N, P, and S, C₅-C₈ aryl, heteroaryl comprising a 5-8 membered ring containing one, two or more heteroatoms selected from 0, N, P, and S, or heteroaryl (C₁-C₄)alkyl, preferably heteroaryl(C₁-C₂)alkyl, the heteroaryl comprising a 5-8 membered ring containing one, two or more heteroatoms selected from 0, N, P, and S, optionally substituted with one or more substituents selected from halogen, C₁-C₄ alkyl, preferably C₁-C₂ alkyl, halogen(C₁-C₄)alkyl, preferably halogen(C₁-C₂)alkyl, C₁-C₄ alkoxy, preferably C₁-C₂ alkoxy, halogen(C₁-C₄)alkoxy, preferably halogen(C₁-C₂)alkoxy, C₁-C₄ alkoxycarbonyl, preferably C₁-C₂ alkoxycarbonyl, heterocyclyl comprising a 5-8 membered ring containing one, two or more heteroatoms selected from O, N, P, and S, di(C₁-C₄)alkylaminocarbonyl, preferably di(C₁-C₂)alkylaminocarbonyl, cyano, nitro, di(C₁-C₄)alkylamino, preferably di-(C₁-C₂)alkylamino, S(O)ₙ-(C₁-C₄)alkyl, preferably S(O)n-(C₁-C₂)alkyl, and S(O)ₙ-halogen(C₁-C₄)alkyl, preferably S(O)ₙ-halogen(C₁-C₂)alkyl;
R¹² represents hydrogen or C₁-C₄ alkyl, preferably C₁-C₂ alkyl, C₃-C₈ cycloalkyl, preferably C₃-C₆ cycloalkyl, C₂-C₄ alkenyl, C₂-C₄ alkynyl, optionally substituted with one or more substituents selected from halogen, C₁-C₄ alkoxy, preferably C₁-C₂ alkoxy, C₁-C₄ alkylmercapto, preferably C₁-C₂ alkylmercapto, cyano, C₁-C₄ alkylsulfinyl, preferably C₁-C₂ alkylsulfinyl, C₁-C₄ alkylsulfonyl, preferably C₁-C₂ alkylsulfonyl, di(C₁-C₄)alkylamino, preferably di(C₁-C₂)alkylamino, C₁-C₄ alkoxycarbonyl, preferably C₁-C₂ alkoxycarbonyl, and di(C₁-C₄)alkylaminocarbonyl, preferably di(C₁-C₂)alkylaminocarbonyl, or represents C₅-C₈ aryl(C₁-C₄)alkyl, heterocyclyl(C₁-C₄)alkyl, preferably heterocyclyl(C₁-C₂)alkyl, the heterocyclyl comprising a 5-8 membered ring containing one, two or more heteroatoms selected from 0, N, P, and S, C₅-C₈ aryl, or heteroaryl comprising a 5-8 membered ring containing one, two or more heteroatoms selected from 0, N, P, and S, optionally substituted with one or more substituents selected from halogen, C₁-C₄ alkyl, preferably C₁-C₂ alkyl, halogen(C₁-C₄)alkyl, preferably halogen(C₁-C₂)alkyl, C₁-C₄ alkoxy, preferably C₁-C₂ alkoxy, halogen(C₁-C₄)alkoxy, preferably halogen(C₁-C₂)alkoxy, C₁-C₄ alkoxycarbonyl, preferably C₁-C₂ alkoxycarbonyl, di(C₁-C₄)alkylaminocarbonyl, preferably di(C₁-C₂)alkylaminocarbonyl, cyano, nitro, di(C₁-C₄)alkylamino, preferably di-(C₁-C₂)alkylamino, S(O)ₙ-(C₁-C₄)alkyl, preferably S(O)ₙ-(C₁-C₂)alkyl, and S(O)ₙ-halogen(C₁-C₄)alkyl, preferably S(O)ₙ-halogen(C₁-C₂)alkyl; and
n is 0, 1 or 2

Further preferred are compounds of formula (Ib) in which R¹ to R⁹ are as defined above for the pre-ferred embodiment of formula (I) and the special group therof.

As used herein,
"alkyl" is defined as a straight or branched C₁₋₁₂ alkyl such as, for example, methyl, ethyl, n- or isopropyl, n-, iso-, sec- or tert-butyl, n-pentyl, n-hexyl, n-heptyl, n-octyl, n-nonyl, n-decyl, n-undecyl, n-dodecyl and the like, preferably, a C₁₋₆ alkyl, more preferably, a C₁₋₄ alkyl.
"alkenyl" is defined as a straight or branched C₂₋₁₂ alkenyl comprising at least one double bond such as, for example, vinyl, allyl, 1-propenyl, isopropenyl, 1-butenyl, 2-butenyl, 3-butenyl, 1,3-butanedienyl, 1-peatenyl, 2-pentenyl, 3-pentenyl, 4-pentenyl, 1,3 pentanedienyl, 1-hemyl, 2-hexsnyl, 3-hexenyl, 4-hexenyl, 5-hexenyl, 1,4-hexanedienyl, and the like, preferably, a C₂₋₆ alkenyl, more preferably a C₂₋₄ alkenyl.
"alkynyl" is defined as a C₂₋₁₂ alkynyl comprising at least one triple bond and optionally further comprising one or more double bonds such as, for example, ethynyl, 1-propynyl, propargyl and the like, preferably, a C₂₋₆ alkynyl, more preferably a C₂₋₄ alkynyl.

As each alkyl portion in "alkoxy", "haloalkyl", "alkoxyalkyl", "alkylaminocarbonyl", "dialkylaminoalkyl", "haloalkoxy", "alkoxycarbonyl", "alkoxycarbonyl" and "alkoxycarbonylalkyl", and the like, the same portions as described in the above-mentioned "alkyl" are exemplified.
"cycloalkyl" is defined as a C₃₋₈ cycloalkyl such as, for example, cyclopropyl, cyclobutyl, cyclopentyl, cyclohexyl, cycloheptyl, cyclooctyl and the like, preferably, a C₃₋₆ cycloalkyl.
""heterocycloalkyl" is defined as a cycloalkyl comprising a 5-8 membered ring containing one, two or more heteroatoms selected from 0, N, P, and S, such as tetrahydrofurane, thiolane, pyrrolidine, oxathiolane, oxazolidine, tetrahydropurane, piperidine, dioxane, and the like, preferred is tetrahydrofurane.
"aryl" is defined as an unsaturated C5-C12 cycloalkyl, such as phenyl, α- or β-naphthyl, preferably, phenyl.
"heteroaryl" is defined as an aryl comprising a 5-8 membered ring containing one, two or more heteroatoms selected from O, N, P, and S, such as furane, thiophene, pyrrole, oxazole, thiazole, pyrazole, pyridine, pyrimidine, pyrazole and the like, preferred are pyrimidine and pyrrazole.
"aralkyl" is defined as, for example, benzyl, phenethyl or α-methylbenzyl, preferably, benzyl.
"halogen" is defined as fluorine, chlorine, bromine or iodine, preferably, fluorine, or chlorine.

As each halogen portion in "haloalkyl", "haloalkoxy", "haloalkylthio", "halogenalkylsulfonyl", "halogenalkylsulfonyloxy", "haloalkenyl", "haloalkynyl", and the like, the same portions as described above are exemplified.

According to a particularly preferred embodiment, I
R¹, R², R³, R⁴, and R⁵ of the formula (I) independently from each other represent hydrogen, halogen, alkyl, alkoxy, haloalkyl, alkoxyalkyl, cycloalkyl, haloalkoxy, alkylthio, haloalkylthio, alkylsulfonyl, alkylsulfonyloxy, halogenalkylsulfonyl, alkoxycarbonyl, acetyl, alkylcarbonyl, alkenylcarbonyl, dialkylamino, cycloalkylamino, alkenyl, haloalkenyl, alkynyl, haloalkynyl, cyano, or nitro;
R⁶ and R⁷ independently from each other represent hydrogen, alkyl, haloalkyl, cycloalkyl, alkoxyalkyl, alkylmercaptoalkyl, alkenyl, or alkynyl, or independently from each other represent aryl, optionally substituted with one or more substituents selected from halogen, alkyl, alkoxy, nitro and cyano;
R⁸ represents -C(Z)NR¹¹R¹²;
Z represents O or S;
R⁹ represents hydrogen, or alkyl;
R¹¹ represents C₁-C₂ alkyl, substituted with one or more substituents selected from halogen, alkoxy, alkylmercapto, alkoxycarbonyl, alkylsulfinyl, alkylsulfonyl and cyano, or represents C₃-C₈ alkyl, cycloalkyl, cycloalkyl, alkenyl, or alkynyl, optionally substituted with one or more substituents selected from halogen, haloalkyl, alkoxy, alkylmercapto, alkylsulfinyl, alkylsulfonyl, dialkylamino, alkoxycarbonyl, and dialkylaminocarbonyl, or represents arylalkyl, heterocyclylalkyl, aryl, heteroaryl, or heteroarylalkyl, optionally substituted with one or more substituents selected from halogen, alkyl, halogenalkyl, alkoxy, halogenalkoxy, alkoxycarbonyl, heterocyclyl, dialkylaminocarbonyl, cyano, nitro, dialkylamino, S(O)ₙ-alkyl, and S(O)ₙ-halogenalkyl;
R¹² represents hydrogen or alkyl, cycloalkyl, alkenyl, alkynyl, optionally substituted with one or more substituents selected from halogen, alkoxy, alkylmercapto, cyano, alkylsulfinyl, alkylsulfonyl, dialkylamino, alkoxycarbonyl, and dialkylaminocarbonyl, or represents arylalkyl, heterocyclylalkyl, aryl, or heteroaryl, optimally substituted with one or more substituents selected from halogen, alkyl, halogenalkyl, alkoxy, halogenalkoxy, alkoxycarbonyl, dialkylaminocarbonyl, cyano, nitro, dialkylamino, S(O)ₙ-alkyl, and S(O)ₙ-halogenalkyl,
n is 0, 1 or 2

According to a special group of compounds of the particularly preferred embodiment defined above,
R¹, R², R³, R⁴, and R⁵ of formula (1) independently from each other represent hydrogen, halogen, preferably chlorine or fluorine, C₁-C₄ alkyl, preferably C₁-C₂ alkyl, C₁-C₄alkoxy, preferably C₁-C₂ alkoxy, C₁-C₄ haloalkyl, preferably C₁-C₂ haloalkyl, (C₁-C₄)alkoxy(C₁-C₄)alkyl, preferably (C₁-C₂)alkoxy(C₁-C₂)alkyl, C₃-C₈ cycloalkyl, preferably C₃-C₆ cycloalkyl, halo(C₁-C₄)alkoxy, preferably halo(C₁-C₂)alkoxy, C₁-C₄ alkylthio, preferably C₁-C₂ alkylthio, halo(C₁-C₄)alkylthio, preferably halo(C₁-C₂)alkylthio, C₁-C₄ alkylsulfonyl, preferably C₁-C₂ alkylsulfonyl, C₁-C₄ alkylsulfonyloxy, preferably C₁-C₂ alkylsulfonyloxy, halogen(C₁-C₄)alkylsulfonyl, preferably halogen(C₁-C₂)alkylsulfonyl, C₁-C₄ alkoxycarbonyl, preferably C₁-C₂ alkoxycarbonyl, acetyl, C₁-C₄ alkylcarbonyl, preferably C₁-C₂ alkylcarbonyl, C₂-C₄ alkenylcarbonyl, di(C₁-C₄)alkylamino, preferably di(C₁-C₂)alkyamino, C₃-C₈ cycloalkylamino, preferably C₃-C₆ cycloalkylamino, C₂-C₄ alkenyl, halo(C₂-C₄)alkenyl, C₂-C₄ alkynyl halo(C₂-C₄)alkynyl, cyano, or nitro;
R⁶ and R⁷ independently from each other represent hydrogen, C₁-C₄alkyl, preferably C₁-C₂ alkyl, C₁-C₄haloatkyl, preferably C₁-C₂ haloalkyl, C₃-C₈ cycloalkyl, (C₁-C₄)alkoxy(C₁-C₄)alkyl preferably (C₁-C₂)alkoxy(C₁-C₂)alkyl, C₁-C₄ alkylmercapto(C₁-C₄)alkyl, preferably C₁-C₂ alkylmercapto(C₁-C₂)alkyl, C₂-C₄alkenyl, or C₂-C₄ alkynyl, or represent independently from each other C₅-C₈ aryl, preferably C₅-C₆ aryl, optionally substituted with one or more substituents selected from halogen, alkyl, alkoxy, nitro and cyano;
R⁸ represents -C(Z)NR¹¹R¹²;
Z represents O or S;
R⁹ represents hydrogen, or C₁-C₄ alkyl, preferably C₁-C₂ alkyl;
R¹¹ represents C₁-C₂ alkyl, substituted with one or more substituents selected from halogen, C₁-C₄ alkoxy, preferably C₁-C₂ alkoxy, C₁-C₄ alkylmercapto, preferably C₁-C₂ alkylmercapto, C₁-C₄ alkoxycarbonyl, preferably C₁-C₂ alkoxycarbonyl, C₁-C₄ alkylsulfinyl, preferably C₁-C₂ alkylsulfinyl, C₁-C₄ alkylsulfonyl, preferably C₁-C₂ alkylsulfonyl, and cyano, or represents C₃-C₈ alkyl, C₃-C₆ cycloalkyl, (C₃-C₆) cycloalkyl(C₁-C₄)alkyl, C₂-C₄ alkenyl, or C₂-C₄ alkynyl, optionally substituted with one or more substituents selected from halogen, halo(C₁-C₄)alkyl, preferably halo(C₁-C₂)alkyl, C₁-C₄ alkoxy, preferably C₁-C₂alkoxy, C₁-C₄ alkylmercapto, preferably C₁-C₂ alkylmercapto, C₁-C₄ alkylsulfinyl, preferably C₁-C₂ alkylsulfinyl, C₁-C₄ alkylsufonyl, preferably C₁-C₂ alkylsulfonyl, di(C₁-C₄)alkylamino, preferably di(C₁-C₂)alkylamino, C₁-C₄ alkoxycarbonyl, preferably C₁-C₂ alkoxycarbonyl, and di(C₁-C₄)alkylaminocarbonyl, preferably di(C₁-C₂)alkylaminocarbonyl, or represents C₅-C₈ aryl(C₁-C₄)alkyl, preferably C₅-C₆ aryl(C₁-C₂)alkyl, heterocyclyl(C₁-C₄)alkyl, preferably heterocyclyl(C₁-C₂)alkyl, the heterocyclyl comprising a 5-8 membered ring containing one, two or more heteroatoms selected from O, N, P, and S, C₅-C₈ aryl, heteroaryl comprising a 5-8 membered ring containing one, two or more heteroatoms selected from O, N, P, and S, or heteroaryl(C₁-C₄)alkyl, preferably heteroaryl(C₁-C₂)alkyl, the hetetoaryl comprising a 5-8 membered ring containing one, two or more heteroatoms selected from O, N, P, and S, optionally substituted with one or more substituents selected from halogen, C₁-C₄ alkyl, preferably C₁-C₂ alkyl, halogen(C₁-C₄) alkyl, preferably halogen(C₁-C₂)alkyl, C₁-C₄ alkoxy, preferably C₁-C₂ alkoxy, halogen(C₁-C₄)alkoxy, preferably halogen(C₁-C₂)alkoxy, C₁-C₄ alkoxycarbonyl, preferably C₁-C₂ alkoxycarbonyl, heterocyclyl comprising a 5-8 membered ring containing one, two or more heteroatoms selected from O, N, P, and S, di(C₁-C₄)alkylaminocarbonyl, preferably di(C₁-C₂)alkylaminocarbonyl, cyano, nitro, di(C₁-C₄)alkylamino, preferably di-(C₁-C₂)alkylamino, S(O)ₙ-(C₁-C₄)alkyl, preferably S(O)ₙ-(C₁-C₂)alkyl, and S(O)ₙ-halogen(C₁-C₄)alkyl, preferably S(O)ₙ-halogen(C₁-C₂)alkyl;
R¹² represents hydrogen or C₁-C₄ alkyl, preferably C₁-C₂ alkyl, C₃-C₈ cycloalkyl, preferably C₃-C₆ cycloalkyl, C₂-C₄ alkenyl, C₂-C₄ alkynyl, optionally substituted with one or more substituents selected from halogen, C₁-C₄ alkoxy, preferably C₁-C₂ alkoxy, C₁-C₄ alkylmercapto, preferably C₁-C₂ alkylmercapto, cyano, C₁-C₄ alkylsulfinyl, preferably C₁-C₂ alkylsulfinyl, C₁-C₄ alkylsulfonyl, preferably C₁-C₂ alkylsulfonyl, di(C₁-C₄)alkylamino, preferably di(C₁-C₂)alkylamino, C₁-C₄ alkoxycarbonyl, preferably C₁-C₂ alkoxycarbonyl, and di(C₁-C₄)alkylaminocarbonyl, preferably di(C₁-C₂)alkylaminocarbonyl, or represents C₅-C₈ aryl(C₁-C₄)alkyl, heterocyclyl(C₁-C₄)alkyl, preferably hetezocyclyl(C₁-C₂)alkyl, the heterocyclyl comprising a 5-8 membered ring containing one, two or more heteroatoms selected from 0, N, P, and S, C₅-C₈ aryl, heteroaryl comprising a 5-8 membered ring containing one, two or more heteroatoms selected from 0, N, P, and S, optionally substituted with one or more substituents selected from halogen, C₁-C₄ alkyl, preferably C₁-C₂ alkyl, halogen(C₁-C₄)alkyl, preferably halogen(C₁-C₂)alkyl, C₁-C₄ alkoxy, preferably C₁-C₂ alkoxy, halogen(C₁-C₄)alkoxy, preferably halogen(C₁-C₂)alkoxy, C₁-C₄ alkoxycarbonyl, preferably C₁-C₂ alkoxycarbonyl, di(C₁-C₄)alkylaminocarbonyl, preferably di(C₁-C₂)alkylaminocarbonyl, cyano, nitro, di(C₁-C₄)alkylamino preferably di-(C₁-C₂)alkylamino, S(O)ₙ-(C₁-C₄)alkyl, preferably S(O)ₙ-(C₁-C₂)alkyl, and S(O)ₙ-halogen(C₁-C₄)alkyl, preferably S(O)ₙ-halogen(C₁-C₂)alkyl; and
n is 0, 1 or 2

Further preferred are compounds of formula (Ib) in which R¹ to R⁹ are as defined above for the particularly preferred embodiment of formula (I) and the special group therof.

According to a very particularly preferred embodiment of the invention,
R¹, R², R³, R⁴, and R⁵ of formula (I) independently from each other represent hydrogen, halogen, alkyl, alkoxy, or haloalkyl;
R⁶ and R⁷ independently from each other represent hydrogen, alkyl, or haloalkyl;
R⁸ represents -C(S)NR¹¹R¹²;
R⁹ represents hydrogen;
R¹¹ represents C₁-C₂ alkyl, substituted with one or more substituents selected from halogen, alkoxy, alkylmercapto, alkoxycarbonyl, alkylsulfinyl, alkylsulfonyl, or cyano, or represents C₃-C₈ alkyl, cycloalkyl, cycloalkylalkyl, or alkenyl, optionally substituted with one or more substituents selected from halogen, haloalkyl, alkoxy, alkylmercapto, alkylsulfinyl alkylsulfonyl, and alkoxycarbonyl, or represents arylalkyl, heterocyclylalkyl, aryl, heteroaryl, or heteroarylalkyl, optionally substituted with one or more substituents selected from halogen, alkyl, halogenalkyl, alkoxy, halogenalkoxy, alkoxycarbonyl, and heterocyclyl;
R¹² represents hydrogen;

According to a special group of compounds of the very particularly preferred embodiment defined above,
R¹, R², R³, R⁴, and R⁵ of formula (I) independently from each other represent hydrogen, halogen, preferably chlorine or fluorine, C₁-C₄ alkyl, preferably C₁-C₂ alkyl, C₁-C₄ alkoxy, preferably C₁-C₂ alkoxy, C₁-C₄ haloalkyl, preferably C₁-C₂ haloalkyl;
R⁶ and R⁷ independently from each other represent hydrogen, C₁-C₄ alkyl, preferably C₁-C₂ alkyl, C₁-C₄ haloalkyl, preferably C₁-C₂ haloalkyl;
R⁸ represents -C(S)NR¹¹R¹²;
R⁹ represents hydrogen;
R¹¹ represents C₁-C₂ alkyl, substituted with one or more substituents selected from halogen, C₁-C₄ alkoxy, preferably C₁-C₂ alkoxy, C₁-C₄ alkylmercapto, preferably C₁-C₂ alkylmercapto, C₁-C₄ alkoxycarbonyl, preferably C₁-C₂ alkoxycarbonyl, C₁-C₄ alkylsulfinyl, preferably C₁-C₂ alkylsulfinyl, C₁-C₄ alkylsulfonyl, preferably C₁-C₂ alkylsulfonyl, and cyano, or represents C₃-C₈ alkyl, C₃-C₆ cycloalkyl, (C₃-C₆)cycloalkyl(C₁-C₄)alkyl, C₂-C₄ alkenyl, optionally substituted with one or more substituents selected from halogen, halo(C₁-C₄)alkyl, preferably halo(C₁-C₂)alkyl, C₁-C₄ alkoxy, preferably C₁-C₂ alkoxy, C₁-C₄ alkylmercapto, preferably C₁-C₂ alkylmercapto, C₁-C₄ alkylsulfinyl, preferably C₁-C₂ alkylsulfinyl, C₁-C₄ alkylsulfonyl, preferably C₁-C₂ alkylsulfonyl, C₁-C₄ alkoxycarbonyl, preferably C₁-C₂ alkoxycarbonyl, or represents heterocyclyl(C₁-C₄)alkyl, preferably heterocyclyl(C₁-C₂)alkyl, the heterocyclyl comprising a 5-8 membered ring containing one, two or more heteroatoms selected from O, N, P, and S, or C₅-C₈ aryl; and
R¹² represents hydrogen.

Further preferred are compounds of formula (Ib) in which R¹ to R⁹ are as defined above for the very particularly preferred embodiment of formula (I) and the special group therof.

According to the most preferred embodiment of the invention,
R¹, R², R³, R⁴, and R⁵ of formula (I) independently from each other represent hydrogen, halogen, alkyl, alkoxy, or haloalkyl;
R⁶ represents alkyl;
R⁷ represents hydrogen;
R⁸ represents -C(S)NR¹¹R¹²;
R⁹ represents hydrogen;
R¹¹ represents C₁-C₂ alkyl, substituted with one or more substituents selected from halogen, alkoxy, alkylmercapto, alkoxycarbonyl, alkylsulfinyl, alkylsulfonyl, or cyano, or represents C₃-C₈ alkyl, cycloalkyl, cycloalkylalkyl, or alkenyl, optionally substituted with one or more substituents selected from halogen, haloalkyl, alkoxy, alkylmercapto, alkylsulfinyl, alkylsulfonyl, and alkoxycarbonyl, or represents arylalkyl, heterocyclylalkyl, aryl, heteroaryl, or heteroarylalkyl, optionally substituted with one or more substituents selected from halogen, alkyl, halogenalkyl, alkoxy, halogenalkoxy, alkoxycarbonyl,and heterocyclyl; and
R¹² represents hydrogen.

According to a special group of compounds of the most preferred embodiment defined above,
R¹, R², R³, R⁴, and R⁵ of formula (I) independently from each other represent hydrogen, halogen, preferably chlorine or fluorine, C₁-C₄ alkyl, preferably C₁-C₂ alkyl, C₁-C₄ alkoxy, preferably C₁-C₂ alkoxy, C₁-C₄ haloalkyl, preferably C₁-C₂ haloalkyl,
R⁶ represents C₁-C₄ alkyl, preferably C₁-C₂ alkyl,
R⁷ represents hydrogen;
R⁸ represents -C(S)NR¹¹R¹²;
R⁹ represents hydrogen;
R¹¹ represents C₁-C₂ alkyl, substituted with one or more substituents selected from halogen, C₁-C₄ alkoxy, preferably C₁-C₂ alkoxy, C₁-C₄ alkylmercapto, preferably C₁-C₂ alkylmercapto, C₁-C₄ alkoxycarbonyl, preferably C₁-C₂ alkoxycarbonyl, C₁-C₄ alkylsulfinyl, preferably C₁-C₂ alkylsulfinyl, C₁-C₄ alkylsulfonyl, preferably C₁-C₂ alkylsulfonyl, and cyano, or represents C₃-C₈ alkyl, C₃-C₆ cycloalkyl, (C₃-C₆)cycloalkyl(C₁-C₄)alkyl, C₂-C₄ alkenyl, optionally substituted with one or more substituents selected from halogen, halo(C₁-C₄)alkyl, preferably halo(C₁-C₂)alkyl, C₁-C₄ alkoxy, preferably C₁-C₂ alkoxy, C₁-C₄ alkylmercapto, preferably C₁-C₂ alkylmercapto, C₁-C₄ alkylsulfinyl, preferably C₁-C₂ alkylsulfinyl, C₁-C₄ alkylsulfonyl, preferably C₁-C₂ alkylsulfonyl, C₁-C₄ alkoxycarbonyl, preferable C₁-C₂ alkoxycarbonyl, or represents heterocyclyl(C₁-C₄)alkyl, preferably heterocyclyl(C₁-C₂)alkyl, the heterocyclyl comprising a 5-8 membered ring containing one, two or more heteroatoms selected from O, N, P, and S, or C₅-C₈ aryl; and
R¹² represents hydrogen.

Further preferred are compounds of formula (Ib) in which R¹ to R⁹ are as defined above for the most preferred embodiment of formula (I) and the special group therof. All compounds of formula (Ib) in which R¹ to R⁹ are as defined above for the most preferred embodiment of formula (I) and the special group therof are present in the S-configruation.

The general or preferred radical definitions or explanations given above apply both to the end products and, correspondingly, to precursors and intermediates. These radical definitions can be combined with one another as desired, i.e. including combinations between the respective preferred ranges.

Preference according to the invention is given to compounds of the formula (I) or (Ib) which contain a combination of the meanings listed above as being preferred.

Particular preference according to the invention is given to compounds of the formula (I) or (Ib) which contain a combination of the meanings listed above as being particularly preferred.

Very particular preference according to the invention is given to compounds of the formula (I) or (Ib) which contain a combination of the meanings listed above as being very particularly preferred.

Most preference according to the invention is given to compounds of the formula (I) or (Ib) which contain a combination of the meanings listed above as being most preferred.

The present invention further relates to a composition comprising at least one compound of the formula (I) or (Ib) as defined in any embodiment above and customary extenders and/or surfactants. Customary extenders surfactants are defined below.

The present invention also relates to a method for controlling pests, wherein a compound of the formula (I) or (Ib) as defined in any embodiment above or a composition comprising at least one compound of the formula (I) or (Ib) as defined in any embodiment above and customary extenders and/or surfactants is allowed to act on the pests and/or their habitat.

Further, the present invention relates to the use of the compounds of the formula (I) or (Ib) as defined in any embodiment above or of compositions comprising at least one compound of the formula (I) or (Ib) as defined in any embodiment above and customary extenders and/or surfactants for controlling pests.

The novel substituted compounds of the formula (I) or (Ib) in which R⁸ is -C(S)NR¹¹R¹², and R¹² is hydrogen are obtained when
a compound of the formula (II) or (IIb) 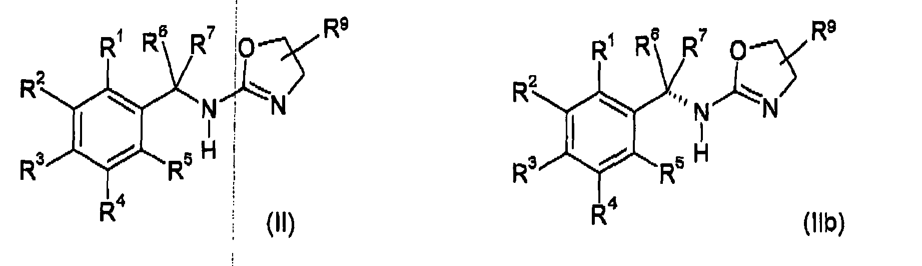 in which
R¹, R², R³, R⁴, R⁵, R⁶, R⁷ and R⁹ are as defined above
   is reacted with a compound of formula (III)

   R¹¹-NCS (III)

   in which
R¹¹ is as defined above,
and N,N-di-isopropylethylamine in an aprotic solvent such as dichloromethane, chloroform, acetonitrile, dimetylformamide, tetrahydrofuran, or ethyl acetate at temperatures from -20 to 120°C, preferably 20-60°C, depending on the solvent. Reaction times are 0.5 to 20 h, ratios of the educts are compound of formula II : compound of formula III of 1: 1 to 1:1.5, preferably 1:1 (process 1),

Furthermore, it has been found that the novel substituted compounds of the formula (I) or (Ib) in which R⁸ is -C(S)NR¹¹R¹², and R¹² is hydrogen can also be obtained when a compound of the formula (II) or (IIb) 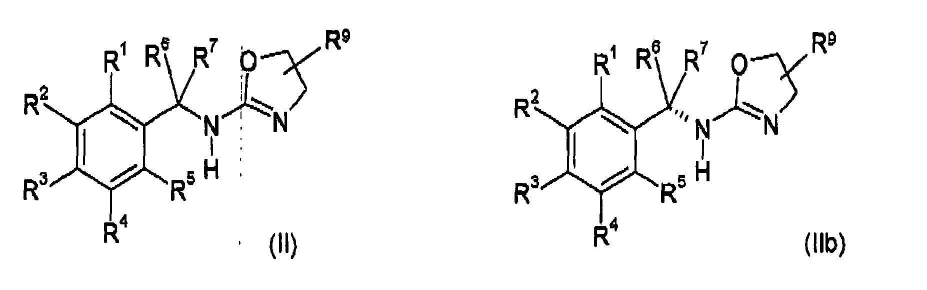 in which
R¹, R², R³, R⁴, R⁵, R⁶, R⁷ and R⁹ are as defined above
   is reacted with thiocarbonyldiimidazole 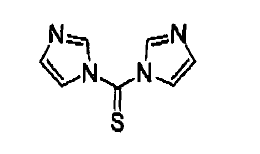 and a compound of the formula (IV)

   R¹¹-NH₂ (IV)

   in which
R¹¹ is as defined above,
in an aprotic solvent such as dichloromethane, chloroform, acetonitrile, dimetylformamide, tetrahy-drofuran, or ethyl acetate at temperatures from -20 to 120°C, preferably 0-40°C, depending on the solvent. Reaction times are 1 to 20 h, ratios of the educts are compound of formula II: thiocarbonyldiimidazole : compound of formula III of 1:1:1 to 1:2:2, preferably 1:1.1:1.1 (process 2).

This method is preferred if an isothiocyanate of an amine is not commercially available (e.g. aminoacetonitrile) and allows the use of the corresponding amine itself to shorten the synthesis pathway.

In general, the synthesis of thioureas by reaction of amines with thiocarbonyldiimidazole is known in the art (cf. e.g. WO 2005/007601, pages 50-52). However, the synthesis of 2-(imino)-1,3-oxazolidine-3-carbothioamides by reaction of an oxazolidine with thiocarbonyldiimidazole and a primary or secondary amine has not been described before. Surprisingly, it was found that this reaction of an oxazolidine with thiocarbonyldiimidazole and a primary or secondary amine is feasible. In view of the low basicity the oxazolidines, this was unexpected for a person skilled in the art.

The amines (IV), thiocarbonyldiimidazole and isothiocyanates (III) are generally known and commercially available.

The compounds of general formulas (II) and (IIb) can be obtained analoguous by methods known from literature (for example WO2006/127426).

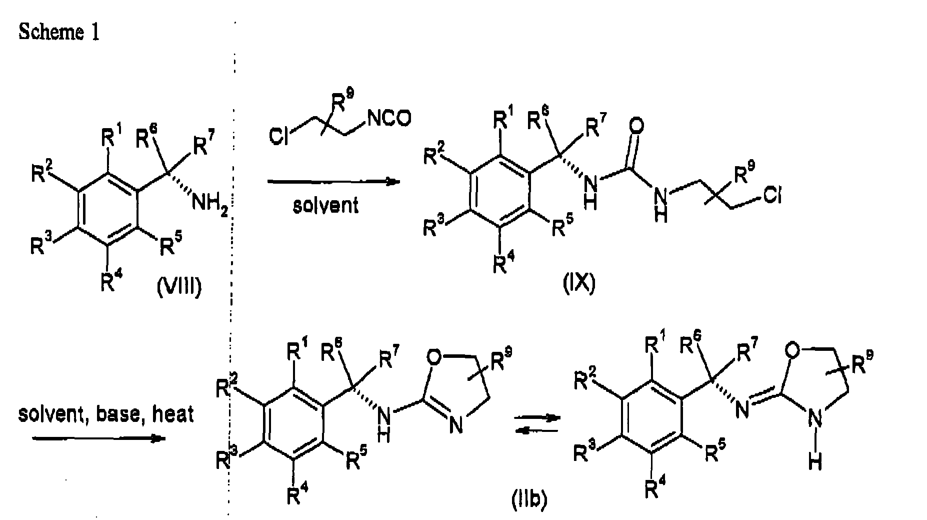

As depicted in scheme I, the reaction of an appropriately substituted benzylamine and an appropriately substituted 2-chloroethyl isocyanate (e,g. in 1,4-dioxane or dichloromethane) yielded the appropriately substituted 1-benzyl-3-(2-chloroethyl)urea, which after heating with a base (e.g. NaOH, H₂O, 1,4-dioxan or diaza(1,3)bicyclo[5.4.0]undecane (DBU), acetonitrile) yielded the appropriately substituted benzyl 1,3-oxazinyl amine (IIb), or (II) if (VIII) is a racemic mixture of appropriately substituted benzylamines. These methods are generally known to those skilled in the art (WO 2006/127426, pages 22, 26, WO2005/063724 page 56, and literature cited therein)

The novel substituted compounds of the formula (I) or (Ib) in which
R⁸ is -C(O)R¹⁰ or -C(O)OR¹⁰ and
R¹² is hydrogen are obtained when
a compound of formula (II) or (IIb) 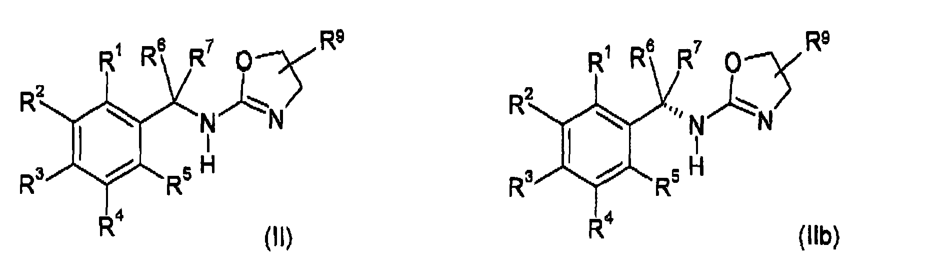 and a compound of formula (V)

X-C(O)R¹⁰ or X-C(O)OR¹⁰ (V)

in which R¹⁰ and R¹¹ are as defined above and X is a leaving group as halogen
or a compound of formula (VI)

-O-C(O)R¹⁰ (VI)

are reacted under basic conditions in an aprotic solvent.

These reactions are generally known to a person skilled in the art. (See WO 2006/127426, page 23 and 32). The compounds of formula (VI) are generally known and commercially available.

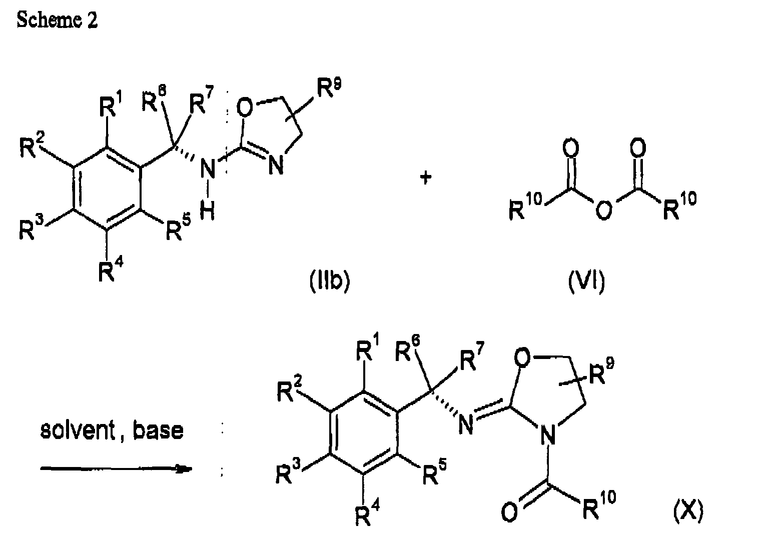

Within this type of reaction depicted in scheme 2, organic as well as inorganic bases are useful (e.g. Diisopropylethylamin, Potassiunicarbonate), preferred are aprotic solvents. The reaction temperatures are between -20 to 40°C, preferred -5°C to 20°C.

Finally, it has been found that the novel compounds of the formula (1) and (Ib) have pronounced biological properties and are suitable especially for controlling animal pests, in particular insects, arachnids and nematodes encountered in agriculture, in forests, in the protection of stored products and in the protection of materials, and also in the hygiene sector.

If appropriate, the compounds of the formula (I) and (Ib) can be present in different polymorphic forms or as a mixture of different polymorphic forms. Both the pure polymorphs and the polymorph mixtures are provided by the invention and can be used according to the invention.

The active compounds according to the invention, in combination with good plant tolerance and favourable toxicity to warm-blooded animals and being tolerated well by the environment, are suitable for protecting plants and plant organs, for increasing the harvest yields, for improving the quality of the harvested material and for controlling animal pests, in particular insects, arachnids, helminths, nematodes and molluscs, which are encountered in agriculture, in horticulture, in animal husbandry, in forests, in gardens and leisure facilities, in the protection of stored products and of materials, and in the hygiene sector. They may be preferably employed as plant protection agents. They are active against normally sensitive and resistant species and against all or some stages of development, The abovementioned pests include:

From the order of the Anoplura (Phthiraptera), for example, Damalinia spp., Haematopinus spp., Linognathus spp., Pediculus spp., Trichodectes spp.

From the class of the Arachnida, for example, Acarus siro, Aceria sheldoni, Aculops spp., Aculus spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

From the class of the Bivalva, for example, Dreissena spp.

From the order of the Chilopoda, for example, Geophilus spp., Scutigera spp.

From the order of the Coleoptera, for example, Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus spp., Lachnosterna consanguinea, Leptmotarsa decemlineata, Lissorhoptrus oryzophilus, Lixus spp., Lyctus spp., Meligethes aeneus, Melolontha melolontha, Migdolus spp., Monochamus spp., Naupactus xauthographus, Niptus hololeucus, Oryctes rhinoceros, Oryzaaphilus surinamensis, Otiorrhynchus sulcatus, Qxycatonia jucunda, Phaodon cochleariae, Phyllophaga spp., Popillia japonica, Premnotrypes spp., Psylliodes chrysocephala, Ptinus spp., Rhizobius ventralis, Rhizopertha dominica, Sitophilus spp., Sphenaphorus spp., Sternechus spp., Symphyletes spp., Tenebrio molitor, Tribolium spp., Trogoderma spp., Tychius spp., Xylotrechus spp., Zabrus spp.

From the order of the Collembola, for example, Onychiurus armatus.

From the order of the Dermaptera, for example, Forficula auricularia.

From the order of the Diplopoda, for example, Blaniulus guttulatus.

From the order of the Diptera, for example, Aedes spp., Anopheles spp., Bibio hortulanus, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp., Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa, Wohlfahrtia spp.

From the class of the Gastropoda, for example, Arion spp., Biomphalaria spp., Bulinus spp., Deroceras spp., Galba spp., Lymnaea spp., Oncomelania spp., Succinea spp.

From the class of the helminths, for example, Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp, Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Namatodirus spp., Oesophagostomum spp., Opisthorchis spp., Onchocerca volvulus, Ostertagia spp., Paragonimus spp., Schistosomen spp., Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

It is furthermore possible to control protozoa, such as Eimeria.

From the order of the Heteroptera, for example, Anasa tristis, Antestiopsis spp., Blissus spp., Calocoris spp., Campylomma livida, Cavelerius spp., Cimex spp,, Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotinophora spp., Stephanitis nashi, Tibraca spp., Triatoma spp.

From the order of the Homoptera, for example, Acyrthosipon spp., Aeneolamia spp., Agonoscena spp., Aleurodes spp., Aleurolobus barodensis, Aleurothrixus spp., Amrasca spp., Anuraphis cardui, Aonidiella spp,, Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlonita onukii, Chromaphis juglandicola, Chrysomphalus ficus, Cicadulina mbila, Cocccmytilus halli, Coccus spp., Cryptomyzus ribis, Dalbulus spp., Dialeurodes spp., Diaphorina spp., Diaspis spp., Doralis spp., Drosicha spp., Dysaphis spp., Dysmicoccus spp., Empoasca spp., Eriosoma spp., Erythroneura spp., Euscelis bilobatus, Geococus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis crysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pimaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala Festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii.

From the order of the Hymenoptera, for example, Diprion spp., Hoplocampa spp., Lasius spp., Monomorium pharaonis, Vespa spp.

From the order of the Isopoda, for example, Armadillidium vulgare, Oniscus asellus, Porcellio seaber.

From the order of the Isoptera, for example, Reticulitermes spp., Odontotermes spp.

From the order of the Lepidoptera, for example, Acronicta major, Aedia leucomelas, Agrotis spp., Alabama argillacea, Anticarsia spp., Barathra brassicae, Bucadatrix thurberiella, Bupalus piniarius, Cacoecia podana, Capua reticulana, Carpocapsa pomonella, Cheimatobia brumata, Chilo spp., Choristoneura fumiferana, Clysia ambiguella, Cnaphalocerus spp., Earias insulana, Ephestia kuehmella, Euproctis chrysorrhoea, Euxoa spp., Feltia spp., Galleria mellonella, Helicoverpa spp., Heliothis spp., Hofmannophila pseodospretella, Homona magnanima, Hyponomeuta padella, Laphygma spp., Lithocolletis blaucardella, Lithophane antennata, Loxagrotis albicosta, Lymantria spp., Malacosoma neustria, Mamestra brassicae, Mocis repanda, Mythimna separata, Oria spp., Oulema oryzae, Panolis flammea, Pectinophora gossypiella, Phyllocnistis citrella, Pieris spp., Plutella xylostella, Prodenia spp., Pseudaletia spp., Pseudoplusia includens, Pyrausta nubilalis, Spodaptera spp., Thermesia gemmatalis, Tinea pellionella, Tineola bisselliella, Tortrix viridana, Trichoplusia spp.

From the order of the Orthoptera, for example, Acheta domesticus, Blatta oriemalis, Blattella germanica, Gryllotalpa spp,, Leucophaea maderae, Locusta spp., Melanoplus spp., Periplaneta americana, Schistocerca gregaria.

From the order of the Siphonaptera, for example, Ceratophyllus spp., Xenopsylla cheopis.

From the order of the Symphyla, for example, Scutigerella immaculata.

From the order of the Thysanoptera, for example, Baliothrips biformis, Enneothrips flavens, Frankliniella spp., Heliothrips spp., Hercinothrips femoralis, Kakothrips spp., Rhipiphorothrips cruentatus, Scirtathrips spp., Taeniothrips cardamoni, Thrips spp.

From the order of the Thysanura, for example, Lepisma saccharina.

The phytoparasitic nematodes include, for example, Anguina spp., Aphelenchoides spp., Belonoaimus spp., Bursaphelenchus spp., Ditylenchus dipsaci, Globodera spp., Heliocotylenchus spp., Heterodera spp., Longidorus spp., Meloidogyne spp., Pratylenchus spp., Radopholus similis, Rotylenchus spp., Trichodorus spp., Tylenchothynchus spp., Tylenchulus spp., Tylenehulus semipenetrans, Xiphinema spp.

If appropriate, the active compounds according to the invention can, at certain concentrations or application rates, also be used as herbicides, safeners, growth regulators or agents to improve plant properties, or as microbicides, for example as fungicides, antimycotics, bactericides, viricides (including agents against viroids) or as agents against MLO (Mycoplasma-like organisms) and RLO (Rickettsia-like organisms). If appropriate, they can also be employed as intermediates or precursors for the synthesis of other active compounds.

The active compounds can be converted to the customary formulations, such as solutions, emulsions, wettable powders, water- and oil-based suspensions, powders, dusts, pastes, soluble powders, soluble granules, granules for broadcasting, suspension-emulsion concentrates, natural materials impregnated with active compound, synthetic materials impregnated with active compound, fertilizers and microencapsulations in polymeric substances.

These formulations are produced in a known manner, for example by mixing the active compounds with extenders, that is liquid solvents and/or solid carriers, optionally with the use of surfactants, that is emulsifiers and/or dispersants and/or foam-formers. The formulations are prepared either in suitable plants or else before or during the application.

Suitable for use as auxiliaries are substances which are suitable for imparting to the active compounds itself and/or to preparations derived therefrom (for example spray liquors, seed dressings) particular properties such as certain technical properties and/or also particular biological properties. Typical suitable auxiliaries are: extenders, solvents and carriers.

Suitable extenders are, for example, water, polar and non-polar organic chemical liquids, for example from the classes of the aromatic and non-aromatic hydrocarbons (such as paraffins, alkylbenzenes, alkylnaphthalenes, chlorobenzenes), the alcohols and polyols (which, if appropriate, may also be substituted, etherified and/or esterified), the ketones (such as acetone, cyclohexanone), esters (including fats and oils) and (poly)ethers, the unsubstituted and substituted amines, amides, lactams (such as *N*-alkylpyrrolidones) and lactones, the sulphones and sulphoxides (such as dimethyl sulphoxide).

If the extender used is water, it is also possible to employ, for example, organic solvents as auxiliary solvents. Essentially, suitable liquid solvents are: aromatics such as xylene, toluene or alkylnaphthalenes, chlorinated aromatics and chlorinated aliphatic hydrocarbons such as chlorobenzenes, chloroethylenes or methylene chloride, aliphatic hydrocarbons such as cyclohexane or paraffins, for example petroleum fractions, mineral and vegetable oils, alcohols such as butanol or glycol and also their ethers and esters, ketones such as acetone, methyl ethyl ketone, methyl isobutyl ketone or cyclohexanone, strongly polar solvents such as dimethyl sulphoxide, and also water.

Suitable solid carriers are:
for example, ammonium salts and ground natural minerals such as kaolins, clays, talc, chalk, quartz, attapulgite, montmorillonite or diatomaceous earth, and ground synthetic minerals, such as finely divided silica, alumina and silicates; suitable solid carriers for granules are: for example, crushed and fractionated natural rocks such as calcite, marble, pumice, sepiolite and dolomite, and also synthetic granules of inorganic and organic meals, and granules of organic material such as paper, sawdust, coconut shells, maize cobs and tobacco stalks; suitable emulsifiers and/or foam-formers are: for example, nonionic and anionic emulsifiers, such as polyoxyethylene fatty acid esters, polyoxyethylene fatty alcohol ethers, for example alkylaryl polyglycol ethers, alkylsulphonates, alkyl sulphates, arylsulphonates and also protein hydrolysates; suitable dispersants are nonionic and/or ionic substances, for example from the classes of the alcohol-POE and/or -POP ethers, acid and/or POP-POE esters, alkyl aryl and/or POP-POE ethers, fat and/or POP-POE adducts, POE- and/or POP-polyol derivatives, POE- and/or POP-sorbitan- or - sugar adducts, alkyl or aryl sulphates, alkyl- or arylsulphonates and alkyl or aryl phosphates or the corresponding PO-ether adducts. Furthermore, suitable oligo- or polymers, for example those derived from vinylic monomers, from acrylic acid, from EO and/or PO alone or in combination with, for example, (poly)alcohols or (poly)amines. It is also possible to employ lignin and its sulphonic acid derivatives, unmodified and modified celluloses, aromatic and/or aliphatic sulphonic acids and their adducts with formaldehyde.

Tackifiers such as carboxymethylcellulose and natural and synthetic polymers in the form of powders, granules or latices, such as gum arabic, polyvinyl alcohol and polyvinyl acetate, as well as natural phospholipids such as cephalins and lecithins, and synthetic phospholipids, can be used in the formulations.

It is possible to use colorants such as inorganic pigments, for example iron oxide, titanium oxide and Prussian Blue, and organic dyestuffs, such as alizarin dyestuffs, azo dyestuffs and metal phthalocyanine dyestuffs, and trace nutrients such as salts of iron, manganese, boron, copper, cobalt, molybdenum and zinc.

Other possible additives are perfumes, mineral or vegetable, optionally modified oils, waxes and nutrients (including trace nutrients), such as salts of iron, manganese, boron, copper, cobalt, molybdenum and zinc.

Stabilizers, such as low-temperature stabilizers, preservatives, antioxidants, light stabilizers or other agents which improve chemical and/or physical stability may also be present.

The formulations generally comprise between 0.01 and 98% by weight of active compound, preferably between 0.5 and 90%.

The active compound according to the invention can be used in its commercially available formulations and in the use forms, prepared from these formulations, as a mixture with other active compounds, such as insecticides, attractants, sterilizing agents, bactericides, acaricides, nematicides, fungicides, growth-regulating substances, herbicides, safeners, fertilizers or semiochemicals.

Particularly favourable mixing components are, for example, the following compounds:
Fungicides:
Inhibitors of nucleic acid synthesis
   benalaxyl, benalaxyl-M, bupirimate, chiralaxyl, clozylacon, dimethirimol, ethirimol, furalaxyl, hymexazol, metalaxyl, metalaxyl-M, ofurace, oxadixyl, oxolinic acid
   Inhibitors of mitosis and cell division
   benomyl, carbendazim, diethofencarb, fuberidazole, pencycuron, thiabendazole, thiophanat-methyl, zoxamide
Inhibitors of respiratory chain complex I
   diflumetorim
Inhibitors of respiratory chain complex II
   boscalid, carboxin, fenfuram, flutolanil, furametpyr, mepronil, oxycarboxin, penthiopyrad, thifluzamide
Inhibitors of respiratory chain complex III
   azoxystrobin, cyazofamid, dimoxystrobin, enestrobin, famoxadone, fenamidone, fluoxastrobin, kresoximmethyl, metominostrobin, orysastrobin, pyraclostrobin, picoxystrobin
   Decouplers
   diaocap, fluazinam
Inhibitors of ATP production
   fentin acetate, fentin chloride, fentin hydroxide, silthiofam
Inhibitors of amino acid biosynthesis and protein biosynthesis
   andoprim, blasticidin-S, cyprodinil, kasugamycin, kasugamycin hydrochloride hydrate, mepanipyrim, pyrimethanil
Inhibitors of signal transduction
   fenpiclonil, fludioxonil, quinoxyfen
Inhibitors of lipid and membrane synthesis
   chlozolinate, iprodione, procymidone, vinclozolin
   ampropylfos, potassium-ampropylfos, edifenphos, iprobenfos (IBP), isoprothiolane, pyrazophos
   tolclofos-methyl, biphenyl
   iodocarb, propamocarb, propamocarb hydrochloride
Inhibitors of ergosterol biosynthesis
   fenhexamid,
   azaconazole, bitertanol, bromuconazole, cyprocanazole, diclobutrazole, difenoconazole, diniconazole, diniconazole-M, epoxiconazole, etaconazole, fenbuconazole, fluquinconazole, flusilazole, flutriafol, furconazole, furconazole-cis, hexaconazole, imibenconazole, ipconazole, metconazole, myclobutanil, paclobutrazole, penconazole, propiconazole, prothioconazole, simeconazole, tebuconazole, tetraconazole, triadimefon, triadimenol, triticonazole, uniconazole, voriconazole, imazalil, imazalil sulphate, oxpoconazole, fenarimol, flurprimidole, nuarimol, pyrifenox, triforine, pefurazoate, prochloraz, triflumizole, viniconazole,
   aldimorph, dodemorph, dodemorph acetate, fenpropimorph, tridemorph, fenpropidin, spiroxamiae,
   naftifine, pyributicarb, terbinafine
Inhibitors of cell wall synthesis
   benthiavalicarb, bialaphos, dimethomorph, flumorph, iprovalicarb, polyoxins, polyoxorim, validamycin A
Inhibitors of melanin biosynthesis
   capropamid, diclocymet, fenoxanil, phthalid, pyroquilon, tricyclazole
   Resistance inductors
   acibenzolar-S-methyl, probenazole, tiadinil
   Multisite
   captafol captan, chlorothalonil, copper salts such as: copper hydroxide, copper naphthenate, copper oxychloride, copper sulphate, copper oxide, oxine-copper and Bordeaux mixture, dichlofluanid, dithianon, dodine, dodine free base, ferbam, folpet, fluorofolpet, guazatine, guazatine acetate, iminoctadine, iminoctadine albesilate, iminoctadine triacetate, mancopper, mancozeb, maneb, metiram, metiram zinc, propineb, sulphur and sulphur preparations containing calcium polysulphide, thiram, tolylfluanid, zineb, ziram

Unknown mechanism
   amibromdol, benthiazol, bethoxazin, capsimycin, carvone, chinomethionat, chloropicrin, cufraneb, cyflufenamid, cymoxanil, dazomet, debacarb, diclomezine, dichlorophen, dicloran, difenzoquat, difenzoquat methyl sulphate, diphenylamine, ethaboxam, ferimzone, flumetover, flusulphamide, fluopicolide, fluoroimide, hexachlorobenzene, 8-hydroxyquinoline sulphate, irumamycin, methasulphocarb, metrafenone, methyl isothiocyanate, mildiomycin, natamycin, nickel dimethyl dithiocarbamate, nitrothal-isopropyl, octhilinone, oxamocarb, oxyfenthiin, pentachlorophenol and salts, 2-phenylphsnol and salts, piperalin, propanosine-sodium, proquinazid, pyrrol nitrin, quintozene, tecloftalam, tecnazene, triazoxide, trichlamide, zarilamid and 2,3,5,6-tetrachloro-4-(methylsulphonyl)pyridime, *N*-(4-chloro-2-nitrophenyl)-*N*-ethyl-4-methylbenzenesulphonamide, 2-amino-4-methyl-*N*-phenyl-5-thiazolecarboxamide, 2-chloro-*N*-(2,3-dihydro-1,1,3-trimethyl-1H-inden-4-yl)-3-pyridinecarbxamide, 3-[5-(4-chlorophenyl)-2,3-dimethylisoxazolidin-3-yl]pyridine, cis-1-(4-chlorophenyl)-2-(1H-1,2,4-triazol-1-yl)cycloheptanol, 2,4-dihydro-5-methoxy-2-methyl-4-[[[[1-[3-(trifluoromethyl)phenyl]ethylidine]amino]oxy]methyl]phenyl]-3H-1,2,3-triazol-3-one (185336-79-2), methyl 1-(2,3-dihydro-2,2-dimethyl-1H-inden-1-yl)-1H-imidazole-5-carboxylate, 3,4,5-trichloro-2,6-pyridinedicarbonitrile, methyl 2-[[[cyclopropyl[(4-methoxyphenyl)immo]methyl]thio]methyl]-.alpha.-(methoxymethylene)benzacetate, 4-chloro-alpha-propynyloxy-*N-*[2-[3-methoxy-4-(2-propynyloxy)phenyl]ethyl]benzacetamide, (2S)-*N*-[2-[4-[[3-(4-chorophenyl)-2-propynyl]oxy]-3-methoxyphenyl]-3-methyl-2-[(methylsulphonyl)amino]butanamide, 5-chloro-7-(4-methylpiperidin-1-yl)-6-(2,4,6-trifluorophenyl)[1,2,4]triazolo[1,5-a]pyrimidine, 5-chloro-6-(2,4,6-trifluorophenyl)-*N-*[(1R)-1,2,2-trimethylpropyl][1,2,4]triazolo[1,5-a]pyrimidin-7-amine, 5-chloro-*N-*[(1R)-1,2-dimethylpropyl]-6-(2,4,6-trifluorophenyl)[1,2,4]triazolo[1,5-a]pyrimidin-7-amine, *N*-[1-(5-bromo-3-chloropyridin-2-yl)ethyl]-2,4-dichloronicotinamide, *N-*(5-bromo-3-chloropyridin-2-yl)methyl-2,4-dichloronicotinamide, 2-butoxy-6-iodo-3-propylbenzopyranon-4-one, *N-*{(Z)-[(cyclopropylmethoxy)-imino][6-(difluoromethoxy)-2,3-difluorophenyl]methyl}-2-benzacetamide, *N-*(3-ethyl-3,5,5-trimethylcyclohexyl)-3-formylamino-2-hydroxybenzamide, 2-[[[[1-[3(1-fluoro-2-phenylethyl)oxy]phenyl]ethylidene]amino]oxy]methyl]-alpha-(methoxyimino)-*N*-methyl-alphaE-benzacetamide, *N*-{2-[3-chloro-5-(trifluoromethyl)pyridin-2-yl]ethyl}-2-(trifluoromethy)benzamide, *N*-(3',4'-dichloro-5-fluorobiphenyl-2-yl)-3-(difluoromethyl)-1-methyl-1H-pyrazole-4-carboxamide, *N*-(6-methoxy-3-pyridinyl)cyclopropanecarboxamide, 1-[(4-methoxyphenoxy)methyl]-2,2-dimethylpropyl-1H-imidazole-1-carboxylic acid, O-[1-[(4-methoxyphenoxy)methyl]-2,2-dimethylpropyl]-1H-imidazole-1-carbothioic acid, 2-(2-{[6-(3-chloro-2-methylphenoxy)-5-fluoropyrimidin-4-yl]oxy}phenyl)-2-(methoxyimino)-*N*-methylacetamide
Bactericides:
   bronopol, dichlorophen, nitrapyrin, nickel dimethyldithiocarbamate, kasugamycin, octhilinone, furancarboxylic acid, oxytetracycline, probenazole, streptomycin, tecloftalam, copper sulphate and other copper preparations.
Insecticides/acsriades/nematicides:
   Acetylcholine esterase (AChE) inhibitors
   carbamates,
   for example alanycarb, aldicarb, aldoxycarb, allyxycarb, aminocarb, bendiocarb, benfuracarb, bufencarb, butacarb, butocarboxim, butoxycarboxim, carbaryl, carbofuran, carbosulphan, cloothocarb, dimetilan, ethiofencarb, fenobucarb, fenothiocarb, formetanate, furathiocarb, isoprocarb, metam-sodium, methiocarb, methomyl, metolcarb, oxamyl, pirimicarb, promecarb, propoxur, thiodicarb, thiofanox, trimethacarb, XMC, xylylcarb, triazamate
organophosphates,
   for example acephate, asamethiphos, azinphos (-methyl, -ethyl), bromophos-ethyl, bromfenvinfos (-methyl), butathiofos, cadusafos, carbophenothion, chlorethoxyfos, chlorfenvinphos, chlormephos, chlorpyrifos (-mothyl/-etyl), coumaphos, cyanofenphos, cyanophos, chlorfenvinphos, demeton-S-methyl, demeton-S-methylsulphone, dialifos, diazinon, dichlofenthion, dichlorvos/DDVP, dicrotophos, dimethoate, dimethylvinphos, dioxabenzofos, disulphoton, EPN, ethion, ethoprophos, etrimfos, famphur, fenamiphos, fenitrothion, fensulphothion, fenhion, flupyrazofos, fonofos, formothion, fosmethilan, fosthiazate, heptenophos, iodofenphos, iprobenfos, isazofos, isufenphos, isopropyl O-salicylate, isoxathion, malathion, mecarbam, methacrifos, methamidophos, methidathion, mevinphos, monocrotophos, naled, omethoate, oxydemeton-methyl, parathion (-methyl/-ethyl), phenthoate, phorate, phosalone, phosmet, phosphamidon, phosphocarb, phoxim, pitimiphos (-methyl/-ethyl), profenofos, propaphos, propetamphos, prothiofos, prothoate, pyraclofos, pyridaphenthion, pyridathion, quinalphos, sebufos, sulphotep, sulprofos, tebupirimfos, temephos, terbufos, totrachlorvinphos, thiometon, triazophos, triclorfon, vamidothion
   Sodium channel modulators I voltage-dependent sodium channel blockers
   pyrethroids,
   for example acrinathrin, allethrin (d-cis-trans, d-trans), beta-cyfluthrin, bifenthrin, bioallethrin, bioallethrin-S-cyclopentyl isomer, bioethanomethrin, biopermethrin, bioresmethrin, chlovaporthrin, cis-cypennethrin, cis-reamethm, cis-permethrin, clocythrin, cycloprothrin, cyfluthrin, cyhalothrin, cypermethrin (alpha-, beta-, theta-, zeta-), cyphenothrin, deltamethrin, empenthrin (1R isomer), esfemvalerate, etofenprox, fenfluthrin, fenpropathrin, fenpyrithrin, fenvalerate, flubrocythrinate, flucythrinate, flufenprox, flumethrin, fluvalinate, fubfenprox, gamma-cyhalothrin, imiprothrin, kadetruin, lambda-cyhalothrin, metofluthrin, permethrin (cis-, trans-), phenothrin (1R-trans-isomer), prallethrin, profluthrin, protrifenbute, pyresmethrin, resmethrin, RU 15525, silafluofen, tau-fluvalinate, tefluthrin, terallethrin, tetramethrin (1R isomer), tralomethrin, transfluthrin, ZXI 8901, pyrethrins (pyrethrum)
DDT
   oxadiazines,
for example indoxacarb
   semicarbazones,
for example metaflumizone (BAS3201)
   Acetylaboline receptor agonists/autagonists
   chloronicotinyls,
for example acetamiprid, clothianidin, dinotefuran, imidacloprid, nitenpyram, nithiazine, thiacloprid, imidaclothiz, AKD-1022, thiamethoxam
   nicotine, bensultap, cartap
Acetylcholine receptor modulators
spinosyns,
   for example spinosad, spinetoram (XDE-175)
   GABA-controlled chloride channel antagonists
   organochlorines,
for example camphechlor, chlordane, endosulphan, gamma-HCH, HCH, heptachlor, lindane, methoxychlor
   fiprols,
for example acetoprole, ethiprole, fipronil, pyrafluprole, pyriprole, vaniliprole
   Chloride channel activators
   mectins,
for example abarmectin, emamectin, emamectin-benzoate, ivermectin, lepimectin, milbemycin
   Juvenile hormone mimetics,
   for example diofenolan, epofenonane, fenoxycarb, hydroprene, kinoprene, methoprene, pyriproxifen, triprene
   Ecdysone agonists/disruptors
   diacylhydrazines,
for example chromafenozide, halofenozide, methoxyfenozide, tebufenozide
   Chitin biosynthesis inhibitors
   benzoylureas,
for example bistrifluron, chlofluazuron, diflubenzuron, fluazuron, flucycloxuron, flufenoxuron, hexaflumuron, lufenuron, novaluron, noviflumuron, penfluron, teflubenzuron, triflumuron
   buprofezin
   cyromazine
   Oxidative phosphorylation inhibitors, ATP disruptors
   diafenthiuron
   organotin compounds,
for example azocyclotin, cyhexatin, fenbutatin-oxide
   Oxidative phosphorylation decouplers acting by interrupting the H-proton gradient
   pyrroles,
for example chlorfenapyr
   dinitrophenols,
   for example binapacyrl, dinobuton, diuocap, DNOC
Site-I electron transport inhibitors
   METIs,
for example fenazaquin, fenpyroximate, pyrimidifen, pyridaben, tebufenpyrad, tolfenpyrad
   hydramethylnon
   dicofol
Site-II electron transport inhibitors
rotenone
Site-III electron transport inhibitors
acequinocyl, fluacrypyrim
Microbial disruptors of the insect gut membrane
Bacillus thuringiensis strains
Lipid synthesis inhibitors
tetronic acids,
for example spirodiclofen, spiromesifen
   tetramic acids,
for example spirotetramat
   carboxamides,
for example flonicamid
   octopaminergic agonists,
for example amitraz
   Inhibitors of magnesium-stimulated ATPase,
   propargite
   nereistoxin analogues,
for example thiocyclam hydrogen oxalate, thiosultap-sodium
   Ryanodin receptor agonists
   benzoic acid dicarboxamides,
for example flubendiamid
   anthronilamides,
for example pynaxypyr (3-bromo-*N*-{4-chloro-2-methyl-6-[(methylamino)carbonyl]phenyl}-1-(3-chloropyridin-2-yl)-1H-pyrazole-5-carboxamide)
   Biologicals, hormones or pheromones
   azadirachtin, Bacillus spec., Beauveria spec., codlemone, Metarrhizium spec., Paecilomyces spec., thuringiensin, Verticillium spec.
   Active compounds with unknown or unspecific mechanisms of action
   fumigants,
for example aluminium phosphide, methyl bromide, sulphuryl fluoride
   antifeedants,
for example cryolite, flonicamid, pymetrozine
   mite growth inhibitors,
for example clofentezine, etoxazole, hexythiazox
   amidoflumet, benclothiaz, benzoximate, bifenazate, bromopropylate, buprofezin, chinomethionat, chlordimeform, chlorobenzitate, chloropicrin, clothiazoben, cycloprene, cyflumetofen, dicyclanil, fenoxacrim, fentrifanil, flubenzimine, flufenerim, flutenzin, gossyplure, hydramethylnone, japonilure, metoxadiazone, petroleum, piperonyl butoxide, potassium oleate, pyridalyl, sulphluramid, tetradifon, tetrasul, triatathene, verbutin

A mixture with other known active compounds, such as herbicides, fertilizers, growth regulators, safeners, semiochemicals, or else with agents for improving the plant properties, is also possible.

When used as insecticides, the active compounds according to the invention can furthermore be present in their commercially available formulations and in the use forms, prepared from these formulations, as a mixture with synergists, Synergists are compounds which increase the action of the active compounds, without it being necessary for the synergistic agent added to be active itself.

When used as insecticides, the active compounds according to the invention can furthermore be present in their commercially available formulations and in the use forms, prepared from these formulations, as a mixture with inhibitors which reduce degradation of the active compound after use in the environment of the plant, on the surface of parts of plants or in plant tissues.

The active compound content of the use forms prepared from the commercially available formulations can vary within wide limits. The active compound concentration of the use forms can be from 0.00000001 to 95% by weight of active compound, preferably between 0.00001 and 1% by weight.

The active compounds are employed in a customary manner appropriate for the use forms.

All plants and plant parts can be treated in accordance with the invention, Plants are to be understood as meaning in the present context all plants and plant populations such as desired and undesired wild plants or crop plants (including naturally occurring crop plants). Crop plants can be plants which can be obtained by conventional plant breeding and optimization methods or by biotechnological and genetic engineering methods or by combinations of these methods, including the transgenic plants and including the plant cultivars protectable or not protectable by plant breeders' rights. Plant parts are to be understood as meaning all parts and organs of plants above and below the ground, such as shoot, leaf, flower and root, examples which may be mentioned being leaves, needles, stalks, stems, flowers, fruit bodies, fruits, seeds, roots, tubers and rhizomes. The plant parts also include harvested material, and vegetative and generative propagation material, for example cuttings, tubers, rhizomes, offshoots and seeds.

Treatment according to the invention of the plants and plant parts with the active compounds is carried out directly or by allowing the compounds to act on the surroundings, habitat or storage space by the customary treatment methods, for example by immersion, spraying, evaporation, fogging, scattering, painting on, injection and, in the case of propagation material, in particular in the case of seeds, also by applying one or more coats.

The active compounds according to the invention are particularly suitable for treating seed, Here, the active compounds according to the invention mentioned above as preferred or particularly preferred may be mentioned as being preferred, Thus, a large part of the damage to crop plants which is caused by pests occurs as early as when the seed is attacked during storage and after the seed is introduced into the soil, during and immediately after germination of the plants. This phase is particularly critical since the roots and shoots of the growing plant are particularly sensitive and even minor damage can lead to the death of the whole plant. Protecting the seed and the germinating plant by the use of suitable active compounds is therefore of particularly great interest.

The control of pests by treating the seeds of plants has been known for a long time and is the subject of continuous improvements. However, the treatment of seed entails a series of problems which cannot always be solved in a satisfactory manner. Thus, it is desirable to develop methods for protecting the seed and the germinating plant which dispense with the additional application of crop protection agents after sowing or after the emergence of the plants. It is furthermore desirable to optimize the amount of active compound employed in such a way as to provide maximum protection for the seed and the germinating plant from attack by pests, but without damaging the plant itself by the active compound employed. In particular, methods for the treatment of seed should also take into consideration the intrinsic insecticidal properties of transgenic plants in order to achieve optimum protection of the seed and the germinating plant with a minimum of crop protection agents being employed.

The present invention therefore in particular also relates to a method for the protection of seed and germinating plants from attack by pests, by treating the seed with an active compound according to the invention. The invention likewise relates to the use of the active compounds according to the invention for the treatment of seed for protecting the seed and the resultant plant from pests. Furthermore, the invention relates to seed which has been treated with an active compound according to the invention so as to afford protection from pests.

One of the advantages of the present invention is that the particular systemic properties of the active compounds according to the invention mean that treatment of the seed with these active compounds not only protects the seed itself, but also the resulting plants after emergence, from pests. In this manner, the immediate treatment of the crop at the time of sowing or shortly thereafter can be dispensed with.

Furthermore, it must be considered as advantageous that the active compounds according to the invention can also be employed in particular in transgenic seed, the plants arising from this seed being capable of expressing a protein directed against pests. By treating such seed with the active compounds according to the invention, certain pests can be controlled merely by the expression of the, for example, insecticidal protein, and additionally be protected by the active compounds according to the invention against damage.

The active compounds according to the invention are suitable for protecting seed of any plant variety as already mentioned above which is employed in agriculture, in the greenhouse, in forests or in horticulture. In particular, this takes the form of seed of maize, peanut, canola, oilseed rape, poppy, soya beans, cotton, beet (for example sugar beet and fodder beet), rice, sorghum and millet, wheat, barley, oats, rye, sunflower, tobacco, potatoes or vegetables (for example tomatoes, cabbage plants). The active compounds according to the invention are likewise suitable for treating the seed of fruit plants and vegetables as already mentioned above. The treatment of the seed of maize, soya beans, cotton, wheat and canola or oilseed rape is of particular importance.

As already mentioned above, the treatment of transgenic seed with an active compound according to the invention is also of particular importance. This takes the form of seed of plants which, as a rule, comprise at least one heterologous gene which governs the expression of a polypeptide with in particular insecticidal properties. In this context, the heterologous genes in transgenic seed may be derived from microorganisms such as Bacillus, Rhizobium, Pseudomonas, Serratia, Trichoderma, Clavibacter, Glomus or Gliocladium. The present invention is particularly suitable for the treatment of transgenic seed which comprises at least one heterologous gone orignating from Bacillus sp. and whose gene product shows activity against the European corn borer and/or the corn root worm. It is particularly preferably a heterologous gene derived from Bacillus thuringiensis.

In the context of the present invention, the active compound according to the invention is applied to the seed either alone or in a suitable formulation, Preferably, the seed is treated in a state which is stable enough to avoid damage during treatment. In general, the seed may be treated at any point in time between harvest and sowing. The seed usually used has been separated from the plant and freed from cobs, shells, stalks, coats, hairs or the flesh of the fruits.

When treating the seed, care must generally be taken that the amount of the active compound according to the invention applied to the seed and/or the amount of further additives is chosen in such a way that the germination of the seed is not adversely affected, or that the resulting plant is not damaged. This must be borne in mind in particular in the case of active compounds which may have phytotoxic effects at certain application rates.

As already mentioned above, it is possible to treat all plants and their parts according to the invention. In a preferred embodiment, wild plant species and plant cultivars, or those obtained by conventional biological breeding methods, such as crossing or protoplast fusion, and parts thereof, are treated. In a further preferred embodiment, transgenic plants and plant cultivars obtained by genetic engineering methods, if appropriate in combination with conventional methods (Genetically Modified Organisms), and parts thereof are treated. The terms "parts", "parts of plants" and "plant parts" have been explained above.

Particularly preferably, plants of the plant cultivars which are in each case commercially available or in use are treated according to the invention. Plant cultivars are to be understood as meaning plants having novel properties ("traits") which have been obtained by conventional breeding, by mutagenesis or by recombinant DNA techniques. These can be cultivars, bio- or genotypes.

Depending on the plant species or plant cultivars, their location and growth conditions (soils, climate, vegetation period, diet), the treatment according to the invention may also result in superadditive ("synergistic") effects. Thus, for example, reduced application rates and/or a widening of the activity spectrum and/or an increase in the activity of the active compounds and compositions which can be used according to the invention, better plant growth, increased tolerance to high or low temperatures, increased tolerance to drought or to water or soil salt content, increased flowering performance, easier harvesting, accelerated maturation, higher harvest yields, higher quality and/or a higher nutritional value of the harvested products, better storage stability and/or processability of the harvested products are possible, which exceed the effects which were actually to be expected.

The transgenic plants or plant cultivars (obtained by genetic engineering) which are preferably to be treated according to the invention include all plants which, by virtue of the genetic modification, received genetic material which imparted particularly advantageous, useful traits to these plants. Examples of such traits are better plant growth, increased tolerance to high or low temperatures, increased tolerance to drought or to water or soil salt content, increased flowering performance, easier harvesting, accelerated maturation, higher harvest yields, higher quality and/or a higher nutritional value of the harvested products, better storage stability and/or processability of the harvested products. Further and particularly emphasized examples of such traits are a better defence of the plants against animal and microbial pests, such as against insects, mites, phytopathogenic fungi, bacteria and/or viruses, and also increased tolerance of the plants to certain herbicidally active compounds, Examples of transgenic plants which may be mentioned are the important crop plants, such as cereals (wheat, rice), maize, soya beans, potatoes, sugar beet, tomatoes, peas and other vegetable varieties, cotton, tobacco, oilseed rape and also fruit plants (with the fruits apples, pears, citrus fruits and grapes), and particular emphasis is given to maize, soya beans, potatoes, cotton, tobacco and oilseed rape. Traits that are emphasized are in particular increased defence of the plants against insects, arachnids, nematodes and slugs and snails by virtue of toxins formed in the plants, in particular those formed in the plants by the genetic material from Bacillus thuringiensis (for example by the genes CryIA(a), CryIA(b), CryIA(c), CryIIA, CryIIIA, CryIIIB2, Cry9c, Cry2Ab, Cry3Bb and CryIF and also combinations thereof) (referred to hereinbelow as "Bt plants"). Traits that are also particularly emphasized are the increased defence of the plants against fungi, bacteria and viruses by systemic acquired resistance (SAR), systemin, phytoalexins, elicitors and resistance genes and correspondingly expressed proteins and toxins. Traits that are furthermore particularly emphasized are the increased tolerance of the plants to certain herbicidally active compounds, for example imidazolinones, sulphonylureas, glyphosate or phosphinotricin (for example the "PAT" gene). The genes which impart the desired traits in question can also be present in combination with one another in the transgenic plants. Examples of "Bt plants" which may be mentioned are maize varieties, cotton varieties, soya bean varieties and potato varieties which are sold under the trade names YIELD GARD® (for example maize, cotton, soya beans), KnockOut® (for example maize), StarLink® (for example maize), Bollgard® (cotton), Nucotn® (cotton) and NewLeaf® (potato). Examples of herbicide-tolerant plants which may be mentioned are maize varieties, cotton varieties and soya bean varieties which are sold under the trade names Roundup Ready® (tolerance to glyphosate, for example maize, cotton, soya bean), Liberty Link® (tolerance to phosphinotricin, for example oilseed rape), IMI® (tolerance to imidazolinones) and STS® (tolerance to sulphonylureas, for example maize). Herbicide-resistant plants (plants bred in a conventional manner for herbicide tolerance) which may be mentioned include the varieties sold under the name Clearfield® (for example maize). Of course, these statements also apply to plant cultivars having these genetic traits or genetic traits still to be developed, which plant cultivars will be developed and/or marketed in the future.

The plants listed can be treated according to the invention in a particularly advantageous manner with the active compounds of the general formula I according to the invention. The preferred ranges stated above for the active compounds also apply to the treatment of these plants. Particular emphasis is given to the treatment of plants with the active compounds specifically mentioned in the present text.

The active compounds according to the invention act not only against plant, hygiene and stored product pests, but also in the veterinary medicine sector against animal parasites (ecto- and endopaxasites), such as hard ticks, soft ticks, mange mites, leaf mites, flies (biting and licking), parasitic fly larvae, lice, hair lice, feather lice and fleas. These parasites include:

From the order of the Anoplurida, for example, Haematopinus spp., Linognathus spp., Pediculus spp., Phtirus spp., Solenopotes spp.

From the order of the Mallophagida and the suborders Amblycerina and Ischnocerina, for example, Trimenopon spp., Menopon spp., Trinoton spp., Bovicola spp., Werneckiella spp., Lepikentron spp., Damalina spp., Trichodectes spp., Felicola spp.

From the order of the Diptera and the suborders Nematocerina and Brachycerina, for example, Aedes spp., Anopheles spp., Culex spp., Simulium, spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrataea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp.

From the order of the Siphonapterida, for example, Pulex spp., Ctenocephalides spp., Xenopsylla spp., Ceratophyllus spp.

From the order of the Heteropterida, for example, Cimex spp., Triatoma spp., Rhodnius spp., Panstrongylus spp.

From the order of the Blattarida, for example, Blatta orientalis, Periplaneta americana, Blattela germanica, Supella spp.

From the subclass of the Acari (Acarina) and the orders of the Meta- and Mesostigmata, for example, Argas spp., Ornithodorus spp., Otobius spp., Ixodes spp., Amblyomma spp., Boophilus spp,, Dermacentor spp., Haemophysalis spp., Hyalomma spp., Rhipicephalus spp., Dermanyssus spp., Raillietia spp., Pneumonyssus spp., Sternostoma spp., Varroa spp.

From the order of the Acanedida (Prostigmata) and Acaridida (Astigmsta), for example, Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp.

The active compounds of the formula (I) according to the invention are also suitable for controlling arthropods which infest agricultural productive livestock, such as, for example, cattle, sheep, goats, horses, pigs, donkeys, camels, buffalo, rabbits, chickens, turkeys, ducks, geese and bees, other pets, such as, for example, dogs, cats, caged birds and aquarium fish, and also so-called test animals, such as, for example, hamsters, guinea pigs, rats and mice. By controlling these arthropods, cases of death and reduction in productivity (for meat, milk, wool, hides, eggs, honey etc.) should be diminished, so that more economic and easier animal husbandry is possible by use of the active compounds according to the invention.

The active compounds according to the invention are used in the veterinary sector and in animal husbandry in a known manner by enteral administration in the form of, for example, tablets, capsules, potions, drenches, granules, pastas, boluses, the feed-through process and suppositories, by parenteral administration, such as, for example, by injection (intramuscular, subcutaneous, intravenous, intraperitoneal and the like), implants, by nasal administration, by dermal use in the form, for example, of dipping or bathing, spraying, pouring on and spotting on, washing and powdering, and also with the aid of moulded articles containing the active compound, such as collars, ear marks, tail marks, limb bands, halters, marking devices and the like.

When used for cattle, poultry, pets and the like, the active compounds of the formula (I) can be used as formulations (for example powders, emulsions, free-flowing compositions), which comprise the active compounds in an amount of 1 to 80% by weight, directly or after 100 to 10 000-fold dilution, or they can be used as a chemical bath.

It has furthermore been found that the active compounds according to the invention also have a strong insecticidal action against insects which destroy industrial materials.

The following insects may be mentioned as examples and as preferred - but without any limitation:
Beetles, such as Hylotrupes bajulus, Chlorophorus pilosis, Anobium punctatum, Xestobium rufovillosum, Ptilinus pecticomis, Dendrobium pertinex, Ernobius mollis, Priobium carpini, Lyctus brunneus, Lyctus africanus, Lyctus planicallis, Lyctus linearis, Lyctus pubescens, Trogoxylon aequale, Minthes rugicollis, Xyleborus spec. Tryptodendron spec. Apate monachus, Bostrychus capucins,
Heterobostrychus brumeus, Sinoxylon spec. Dinoderus minutus;
Hymenopterons, such as Sirex juvencus, Urocerus gigas, Urocerus gigas taignus, Urocerus augur;
Termites, such as Kalotermes flavicollis, Cryptotermes brevis, Heterotermes indicola, Reticulitermes flavipes, Reticulitermes santonensis, Reticulitermes lucifugus, Mastotermas darwiniensis, Zootermopsis nevadensis, Coptotermes formosanus;
Bristletails, such as Lepisma saccharina.

Industrial material in the present connection are to be understood as meaning non-living materials, such as, preferably, plastics, adhesives, sizes, papers and cardboards, leather, wood and processed wood products and coating compositions.

The ready-to-use compositions may, if appropriate, comprise further insecticides and, if appropriate, one or more fungicides.

With respect to possible additional additives, reference may be made to the insecticides and fungicides mentioned above.

The active compounds according to the invention can likewise be employed for protecting objects which come into contact with seawater or brackish water, such as hulls, screens, neks, buildings, moorings and signalling systems, against fouling.

Furthermore, the active compounds according to the invention, alone or in combinations with other active compounds, may be employed as antifouling agents.

In domestic, hygiene and stored-product protection, the active compounds are also suitable for controlling animal pests, in particular insects, arachnids and mites, which are found in enclosed spaces such as, for example, dwellings, factory halls, offices, vehicle cabins and the like. They can be employed alone or in combination with other active compounds and auxiliaries in domestic insecticide products for controlling these pests. They are active against sensitive and resistant species and against all developmental stages. These pests include:
From the order of the Scorpionidea, for example, Buthus occitanus.

From the order of the Acarina, for example, Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.

From the order of the Araneae, for example, Aviculariidae, Araneidae,

From the order of the Opiliones, for example, Pseudoscorpiones chelifer, Pseudoscorpiones cheiridium, Opiliones phalangium.

From the order of the Isopoda, for example, Oniscus asellus, Porcellio scaber.

From the order of the Diplopoda, for example, Blaniulus guttulatus, Polydesmus spp.

From the order of the Chilopoda, for example, Geophilus spp.

From the order of the Zygentoma, for example, Ctenolepisma spp., Lepisma saccharina, Lepismodes inquilinus.

From the order of the Blattaria, for example, Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

From the order of the Saltatoria, for example, Acheta domesticus.

From the order of the Dermaptera, for example, Forficula auricularia.

From the order of the Isoptera, for example, Kalotermes spp., Reticulitermes spp.

From the order of the Psocoptera, for example, Lepinatus spp., Liposcelis spp.

From the order of the Coleoptera, for example, Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

From the order of the Diptera, for example, Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa,

From the order of the Lepidoptera, for example, Achroia grisella, Galleria mellonella, Plodia interpunctella, Tinea cloacella, Tinea pellionella, Tineola bissclliella.

From the order of the Siphonaptera, for example, Ctenocephalides canis, Ctenocephalides felis, Pulex irritans, Tunga penetrans, Xenopsylla cheopis.

From the order of the Hymenoptera, for example, Camponotus herculeanus, Lasius fuliginosus, Lasius niger, Lasius umbratus, Monomorium pharaonis, Paravespula spp,, Tetramorium caespitum.

From the order of the Anoplura, for example, Pediculus humanus capitis, Pediculus humanus corporis, Pemphigus spp., Phylloera vastatrix, Phthirus pubis.

From the order of the Heteroptera, for example, Cimex hemipterus, Cimex lectularius, Rhodinus prolixus, Triatoma infestans.

In the field of household insecticides, the active compounds according to the invention are used alone or in combination with other suitable active compounds, such as phosphoric esters, carbamates, pyrethroids, neonicotinoids, growth regulators or active compounds from other known classes of insecticides.

The active compounds according to the invention are used in aerosols, pressure-free spray products, for example pump and atomizer sprays, automatic fogging systems, foggers, foams, gels, evaporator products with evaporator tablets made of cellulose or polymer, liquid evaporators, gel and membrane evaporators, propeller-driven evaporators, energy-free, or passive, evaporation systems, moth papers, moth bags and moth gels, as granules or dusts, in baits for spreading or in bait stations.

### Preparation Examples:

### Process 1

### (2Z)-2-{[1-(2-chlorophenyl)ethyl]imino}-N-(2-methoxyethyl)-1,3-oxazolidine-3-carbothioamide

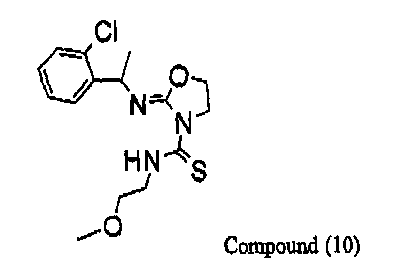

400 mg (1.78 mmol) N-[1-(2-chlorophenyl)ethyl]-4,5-dihydro-1,3-oxazol-2-amine were initially charged in 10 ml dichloromethane with 209 mg (1,78 mmol) methoxyethylisothiocyanate and subsequently, 214 mg (1.66 mmol) N,N-diisopropyletyhlamine, solved in 1 ml dichlormethane, were added tropwise. The reaction mixture was stirred 15 h at 20°C and finally all volatile ingredients were evaporated. The reaction gave 600 mg (99 % of theory) of compound 10. Log P acidic 4.08, 1H-NMR (CDCl₃) q 5.24 ppm.

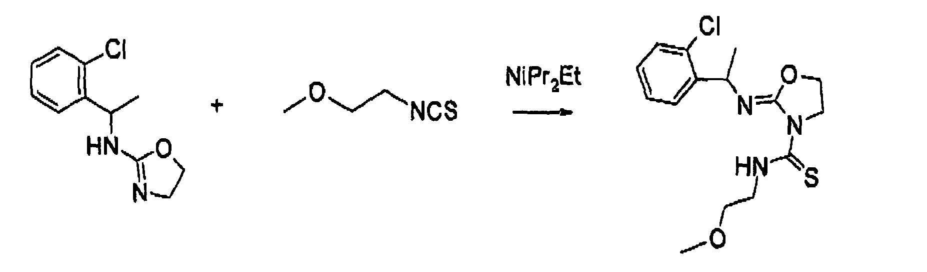

This procedure can be employed analogously for all compounds of formula (I) in which R⁸ is - C(S)NR¹¹R¹², and R¹² is hydrogen. If the oxazole-amine is present as chiral compound according to formula (IIb), this procedure can be employed analogously for all compounds of formula (Ib) in which R⁸ is -C(S)NR¹¹R¹², and R¹² is hydrogen.

### Process 2

### (2Z)-N-(cyanomethyl)-2-[(1-phenylethyl)imino]-1,3-oxazolidine-3-carbothiamide

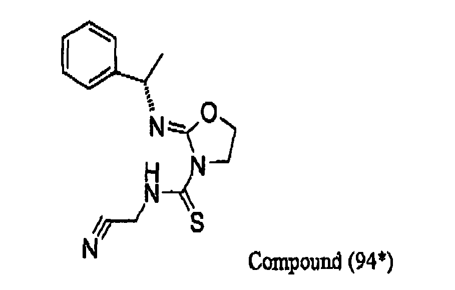

200 mg (1.05 mmol) N-[(1S)-1phenylethyl]-4,5-dihydro-1,3-oxazol-2-amine and 206 mg (1.16 mmol) thiocarbonyldiimidazol were stirred in 40 ml dichloromethane for 1 ½ h at room temperature. Subsequently, 65 mg (1,16 mmol) aminoacetonitrile were added and the mixture was further stirred for 15 h at room temperature, For processing, water was added, the organic phase was removed, dried with sodium sulfate, filtered, and solvents were removed. The product obtained was purified by column chromatography with dichloromethane on aluminium oxide and 50 mg (16 % of theory) of compound 94* were thus isolated. LogP acidic 3.07, 1H-NMR (DMSO-d6) q 4.78 ppm.

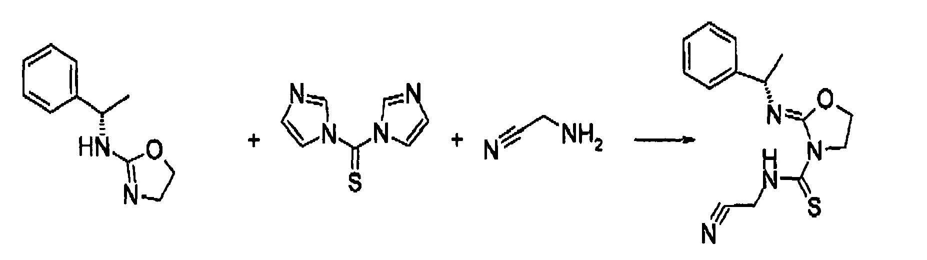

This procedure can be employed analogously for all compounds of formula (Ib) in which R⁸ is - C(S)NR¹¹R¹², and R¹² is hydrogen or alkyl. If the oxazole-amine is present as racemic mixture according to formula (II), this procedure can be employed analogously for all compounds of formula (I) in which R⁸ is -C(S)NR¹¹R¹², and R¹² is hydrogen or alkyl.

Further compounds of the formula (I) and (Ib) are listed in Table 1 below.

**Table 1**

| Compounds of the formula (I) or (Ib) | | | | | | | | | |
|---|---|---|---|---|---|---|---|---|---|
| 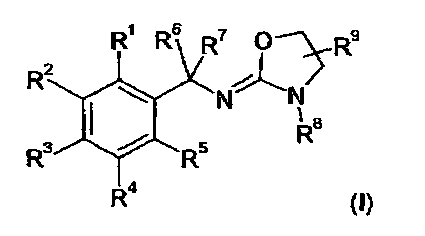 wherein **R**⁹ represents hydrogen. | | | | | 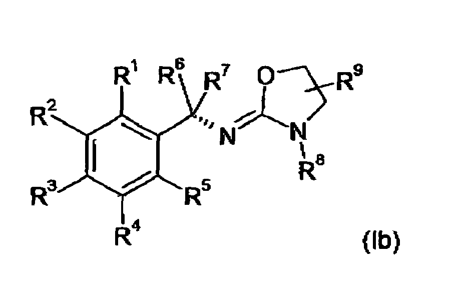 | | | | |
| Phys.Data: ¹H-NMR δ in CDCl₃ (A) or DMSO D6 (B), or log P (acidic) (C) | | | | | | | | | |

| **Compd. No** | **R¹** | **R²** | **R³** | **R⁴** | **R⁵** | **R⁶** | **R⁷** | **R⁸** | **Phys. Data** |
|---|---|---|---|---|---|---|---|---|---|
| 1 | Me | F | H | H | H | H | H | C(S)-NH-CH₂-CH₂-O-Me | 3.72 m (A) |
| 2* | H | H | H | H | H | Me | H | C(S)-NH-CH₂-CH₂-O-Me | 4.8 q (A) |
| 3* | H | H | H | H | H | Me | H | C(S)-NH-CH₂-CF₃ | 4.8 q (A) |
| 4* | H | H | H | H | H | Me | H | C(S)-NH-CH₂-CH₂-S-Me | 5.11 tr (A) |
| 5* | H | H | H | H | H | Me | H | C(S)-NH-CH(CH₃)-CH₂-S-Me R-form | 5.02 tr (B) |
| 6 | H | H | H | H | H | Et | H | C(S)-NH-CH₂-CH₂-O-Me | 4.53 tr (B) |
| 7 | H | H | H | H | H | Et | H | C(S)-NH-CH₂-CF₃ | 4.6 m(B) |
| 8 | H | H | H | H | H | Et | H | C(S)-NH-CH₂-COOEt | 4.53 tr (B) |
| 9 | Cl | H | H | H | H | Me | H | C(S)-NH-CH₂-COOEt | 5.26 q (A) |
| 10 | Cl | H | H | H | H | Me | H | C(S)-NH-CH₂-CH₂-O-Me | 5.24 q (A) |
| 11 | H | Cl | H | H | H | Me | H | C(S)-NH-CH₂-CH₂-O-Me | 4.76 q (A) |
| 12* | F | H | H | H | H | Me | H | C(S)-NH-CH₂-CH₂-O-Me | 513 q (A) |
| 13* | H | F | H | H | H | Me | H | C(S)-NH-CH₂-COOEt | 4.8 q (A) |
| 14 | H | F | H | H | H | Me | H | C(S)-NH-CH₂-CH₂-O-Me | 4.8 q (A) |
| 15* | H | F | H | H | H | Me | H | C(S)-NH-CH₂-CH₂-O-Me | 4.8 q (A) |
| 16* | H | H | H | H | H | Me | H | C(S)-NH-CH₂-CH-(O-Me)₂ | 3.42 (C) |
| 17* | H | H | H | H | H | Me | H | C(S)-NH-CH(CH₃)-n-C₃H₇ | 4.77 (C) |
| 18* | H | H | H | H | H | Me | H | C(S)-NH-CHEt₂ | 4.77 (C) |
| 19* | H | H | H | H | H | Me | H | C(S)-NH-CH(CH₃)-CH(CH₃)₂ | 4.77 (C) |
| 20* | H | H | H | H | H | Me | H | C(S)-NH-CH₂-Pr | 3.99 (C) |
| 21* | H | H | H | H | H | Me | H | C(S)-NH-CH₂-CH=CH₂ | 3.63 (C) |
| 22* | H | H | H | H | H | Me | H | C(S)-NH-n-Pr | 3.84 (C) |
| 23* | H | H | H | H | H | Me | H | C(S)-NH-n-Bn | 4.35 (C) |
| 24* | H | H | H | H | H | Me | H | C(S)-NH-CH(CH₃)-CF₃ | 4.51 (C) |
| 25* | H | H | H | H | H | Me | H | C(S)-NH-CH₂-CH(CH₃)₂ | 4.35 (C) |
| 26* | H | H | H | H | H | Me | H | C(S)-NH-CH₂-CH₂COOMe | 3.15 (C) |
| 27* | H | H | H | H | H | Me | H | C(S)-NH-CH(CH₃)-CH₂-COOEt | 3.94 (C) |
| 28* | H | H | H | H | H | Me | H | C(S)-NH-CH₂-CH₂-COOBt | 3.58 (C) |
| 29* | H | H | H | H | H | Me | H | C(S)-NH-CH(i-Pr)-COOMe | 4.25 (C) |
| 30* | H | H | H | H | H | Me | H | C(S)-NH-CH(CH₃)-COOBt | 3.99 (C) |
| 31* | H | H | H | H | H | Me | H | C(S)-NH-CH(Et)-COOEt | 3.94 (C) |
| 32* | H | H | H | H | H | Me | H | 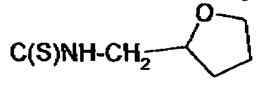 | 3.58 (C) |
| 33* | H | H | H | H | H | Me | H | 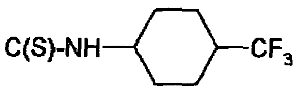 | 4.92 (C) |
| 34* | H | H | H | H | H | Me | H | C(S)-NH-i-Pr | 3.94 (C) |
| 35* | H | H | H | H | H | Me | H | C(S)-NH-c-Pr | 3.37 (C) |
| 36* | H | H | H | H | H | Me | H | C(S)-NH-c-Pentyl | 4.56 (C) |
| 37* | H | H | H | H | H | Me | H | 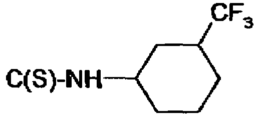 | 5.03 (C) |
| 38* | H | H | H | H | H | Me | H | C(S)-NH-CH₂-Ph | 4.30 (C) |
| 39* | H | H | H | H | H | Me | H | C(S)-NH-CH(CH₃)Ph | 4.67 (C) |
| 40* | H | H | H | H | H | Me | H | 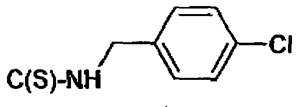 | 4.77 (C) |
| 41* | H | H | H | H | H | Me | H | 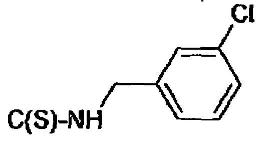 | 4.77 (C) |
| 42* | H | H | H | H | H | Me | H | 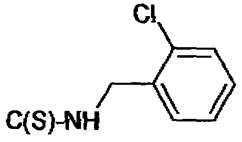 | 4.77 (C) |
| 43* | H | H | H | H | H | Me | H | 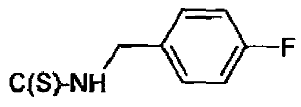 | 4.30 (C) |
| 44* | H | H | H | H | H | Me | H | 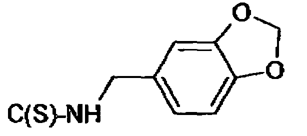 | 3.99 (C) |
| 45* | H | H | H | H | H | Me | H | 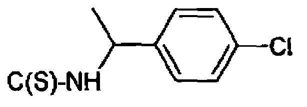 | 5.19 (C) |
| 46* | H | H | H | H | H | Me | H | C(S)-NH-CH₂-CH₂-Ph | 4.51 (C) |
| 47* | H | H | H | H | H | Me | H | 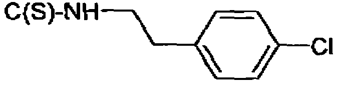 | 4.92 (C) |
| 48* | H | H | H | H | H | Me | H | 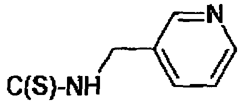 | 2.03 (C) |
| 49* | H | H | H | H | H | Me | H | 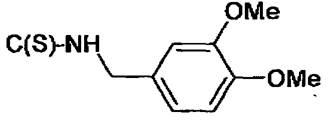 | 3.63 (C) |
| 50* | H | H | H | H | H | Me | H | 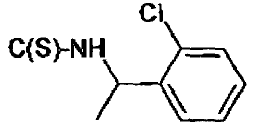 | 5.08 (C) |
| 51* | H | H | H | H | H | Me | H | 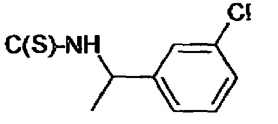 | 5.14 (C) |
| 52* | H | H | H | H | H | Me | H | 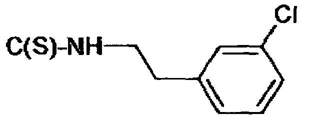 | 4.92(C) |
| 53* | H | H | H | H | H | Me | H | 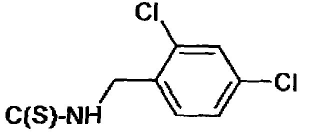 | 5.42 (C) |
| 54* | H | H | H | H | H | Me | H | 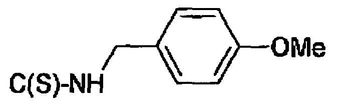 | 4.15 (C) |
| 55* | H | H | H | H | H | Me | H | 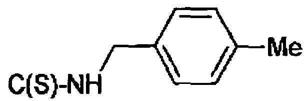 | 4.72 (C) |
| 56* | H | H | H | H | H | Me | H | 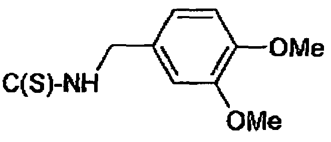 | 3.78 (C) |
| 57 | H | H | H | H | H | Et | H | C(S)-NH-CH(OH₃)₂ | 4.41 (C) |
| 58 | H | H | H | H | H | Et | H | C(S)-NH-c-Pr | 3.85 (C) |
| 59 | H | H | H | H | H | Et | H | C(S)-NH-t-Bu | 5.08 (C) |
| 60 | H | H | H | H | H | Et | H | 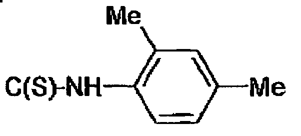 | 5.14(C) |
| 61 | H | H | H | H | H | Et | H | 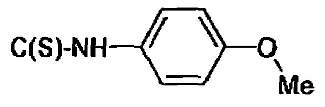 | 4.51 (C) |
| 62 | H | H | H | H | H | Et | H | 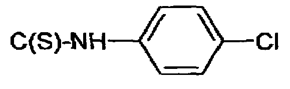 | 5A (C) |
| 63 | H | H | H | H | H | Et | H | 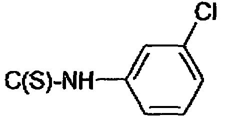 | 5.4 (C) |
| 64 | H | H | H | H | H | Et | H | 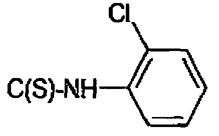 | 4.97 (C) |
| 65 | H | H | H | H | H | Et | H | 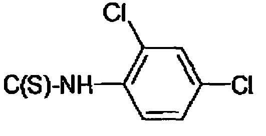 | 5.78 (C) |
| 66 | H | H | H | H | H | Et | H | 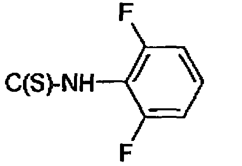 | 4.41 (C) |
| 67 | H | H | H | H | H | Et | H | 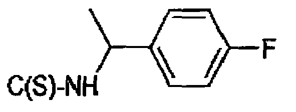 | 5.02 (C) |
| 68 | H | H | H | H | H | Et | H | 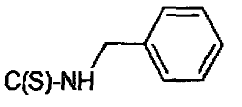 | 4.68 (C) |
| 69 | H | H | H | H | H | Et | H | 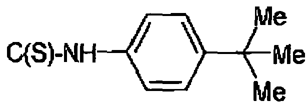 | 6.09 (C) |
| 70 | H | H | H | H | H | Et | H | 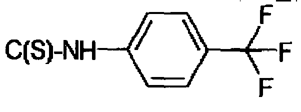 | 5.52(C) |
| 71 | H | H | H | H | H | Et | H | 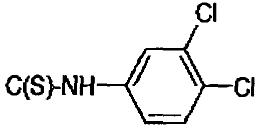 | 6.03 (C) |
| 72 | H | H | H | H | H | Et | H | 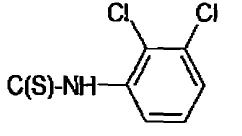 | 5.46 (C) |
| 73 | H | H | H | H | H | Et | H | 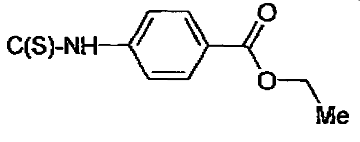 | 5.21 (C) |
| 74 | H | H | H | H | H | Et | H | 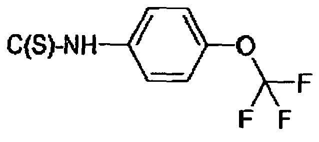 | 5.59 (C) |
| 75 | H | H | H | H | H | Et | H | 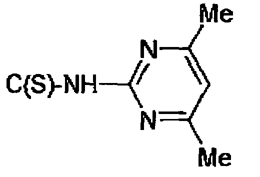 | 2.05 (C) |
| 76 | H | H | H | H | H | Et | H | 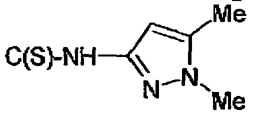 | 3.85 (C) |
| 77 | H | H | H | H | H | Et | H | 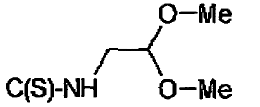 | 3-89 (C) |
| 78 | H | H | H | H | H | Et | H | C(S)-NH-CH(CH₃)-CH₂CH₂CH₃ | 5-31 (C) |
| 79 | H | H | H | H | H | Et | H | C(S)-NH-CH(CH₂CH₃)-CH₂CH₃ | 5.25 (C) |
| 80 | H | H | H | H | H | Et | H | C(S)-NH-CH(CH₃)-CH(CH₃)CH₃ | 5.25 (C) |
| 81 | H | H | H | H | H | Et | H | C(S)-NH-CH₂-c-Pr | 4-51 (C) |
| 82 | H | H | H | H | H | Et | H | C(S)-NH-CH₂-CH(CH₃)CH₃ | 4.91 (C) |
| 83 | H | H | H | H | H | Et | H | C(S)-NH-CH₂-CH=CH₂ | 4-17 (C) |
| 84 | H | H | H | H | H | Et | H | C(S)-NH-CH₂CH₂CH₃ | 4.34 (C) |
| 85 | H | H | H | H | H | Et | H | C(S)-NH-CH₂CH₂CH₂CH₃ | 4.85 (C) |
| 86 | H | H | H | H | H | Et | H | C(S)-NH-CH(CH₃)-CF₃ | 4.97 (C) |
| 87 | H | H | H | H | H | Et | H | C(S)-NH-CH₂CH₂C(O)OCH₃ | 3.61 (C) |
| 88 | H | H | H | H | H | Et | H | C(S)-NH-CH(CH₃)CH₂C(O)Oet | 4.45 (C) |
| 89 | H | H | H | H | H | Et | H | C(S)-NH-CH₂CH₂CH₂C(O)Oet | 4.00 (C) |
| 90 | H | H | H | H | H | Et | H | 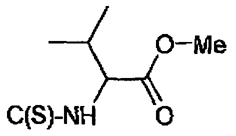 | 4.68 (C) |
| 91 | H | H | H | H | H | Et | H | C(S)-NH-CH(CH₃)-C(O)OCH₂CH₃ | 4.45 (C) |
| 92 | H | H | H | H | H | Et | H | C(S)-NH-CH(CH₂CH₃)-C(O)OCH₃ | 4.40(C) |
| 93 | H | H | H | H | H | Et | H | 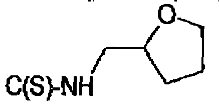 | 4.05(C) |
| 94* | H | H | H | H | H | Me | H | C(S)-NH-CH₂CN | 4.78q(B) |
| 95 | H | F | H | H | H | Et | H | C(S)-NH-CH₂CH₂-O-Me | 4.56 tr (A) |
| 96 | H | F | H | H | H | CF₃ | H | C(S)-NH-CH₂-CH₂-O-Me | |
| 97 | H | H | H | H | H | Ph | H | C(S)-NH-CH₂-CH₂-O-Me | |
| 98* | H | H | H | H | H | Me | H | C(S)-NH-CH₂CH₂-SO₂-Me | |
| 99* | H | H | H | H | H | Me | H | C(S)-NH-CH₂-CH₂-SO-Me | |
| 100* | H | H | H | H | H | Me | H | C(S)-NH-C(CH₃)₂-SO₂-Me | |
| 101 | H | Cl | H | H | H | 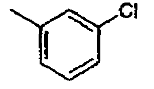 | H | C(S)-NH-CH₂-CH₂-O-Me | |
| 102 | Cl | H | Cl | Cl | H | Me | H | C(S)-NH-CH₂-CH₂-O-Me | |
| 103 | H | F | OMe | H | H | Me | H | C(S)-NH-CH₂-CH₂-O-Me | |
| 104 | H | OMe | H | H | H | Me | H | C(S)-NH-CH₂-CH₂-O-Me | |
| 104 | H | OMe | H | H | H | Me | H | C(-S)-NH-CH₂-CH₂-O-Me | |
| 105 | Me | F | H | H | H | Me | H | C(S)-NH-CH₂-CH₂-O-Me | |
| 106 | OMe | H | OMe | H | H | Me | H | C(S)-NH-CH₂-CH₂-O-Me | |
| 107 | Me | H | Me | H | H | Me | H | C(S)-NH-CH₂-CH₂-O-Me | |
| 108 | Cl | H | Cl | H | H | Me | H | C(S)-NH-CH₂-CH₂-O-Me | |
| 109 | H | CF₃ | H | H | H | Me | H | C(S)-NH-CH₂-CH₂-O-Me | |
| 110 | H | F | H | H | H | Et | H | C(S)-NH₂-CH₂-CH₂-O-Me | 4.55 t(A) |

| | | | | | | | | | |
|---|---|---|---|---|---|---|---|---|---|
| *: S-enantiomers | | | | | | | | | |

### Preparation of starting materials

### N-[1-(2-chlorophenyl)ethyl]-4,5-dihydro-1,3-oxazol-2-amitie

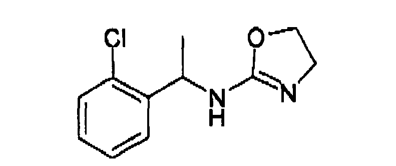

### Step 1:

3,2 g (20.6mmol) 1-(2-chlorophenyl)ethaneamine [CAS 39959-67-6] and 52.3 g (22.6 mmol) triethylamine were given into 20 ml dichloromethane and afterwards 2.4 g (22.6 mmol) 2-chloroethylisocyanat werde added dropwise. The mixture was allowed to react 15 hours at 20°C. The volatile components were evaporated to give 1-(2-chloroethyl)-3-[1-(2-chlorophenyl)ethyl]urea (log p = 2.3) that was used in step 2 without further purification

### Step 2:

7,2 g (27.5 mmol) 1-(2-chloroethyl)-3-[1-(2-chlorophenyl)ethyl]urea, 5.25 g (34.5 mmol) 1,8-di=bicyclo(5.4.0)uudm.7-en (DBU) were heated to 60 °C in 60 ml of acetonitrile for about 15 hours. The volatile components were evaporated, and the residue was dissolved in water and di-chloremethane. The organic phase was dried with sodiumsulfate and after filtration evaporated. The remaining solid was washed with cold acetonitrile to give 2.4 g N-[1-(2-chlorophenyl)ethyl]-4,5-dihydro-1,3-oxazol-2-amine (39 % of theory). (1H-NMR (CDCl3): 4.76 quartett)

### N-[(1S)-1-phenylethyl]4,5-dihydro-1,3-oxazol-2-amine

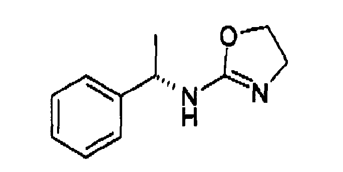

### Step 1:

6,1 g (50,3 mmol) 1(S)-1-phenylethylamine [CAS 2627-86-3] and 5.6 g (55.3 mmol) triethylamine were given into 45 ml dichloromethane and afterwards 5.8 g (22.6 mmol) 2-cblaroethylisocyanate were added dropwise, The mixture was allowed to react 15 hours at 20°C. The volatile components were evaporated to give 1-(2-chloroethyl)-3-[(1S)-1-phenylethyl]urea (log p =1,76) that was used in step 2 without further purification,

### Step 2:

11.4 g (50.3 mmol) 1-(2-chloroethyl)-3-[(1S)-1-phenylcthyl]urea, and 9.6 g (63 mmol) 1,8-diazabicyclo(5,4,0)undec-7-en (DBU) were heated to 60°C in 50 ml of acetonitrile for about 15 hours. The volatile components were evaporated; the residue was dissolved in ethyl acetate and washed three times with water. The organic phase was dried with sodiumsulfate and after filtration evaporated to give 8 g N-[(1S)-1-phenylethyl]-4,5-dihydro-1,3-oxazol-2-amine (79 % of theory). (1H-NMR (DMSO-d6): 4.61 quartett)

## Claims

1. Compounds of the formula (I) 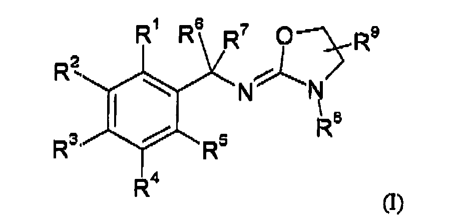 in which
R¹, R², R³, R⁴, and R⁵ independently from each other represent hydrogen, halogen, hydroxy, alkyl, alkoxy, haloalkyl, alkoxyalkyl, cycloalkyl, cyanoalkyl, haloalkoxy, alkylthio, haloalkylthio, alkylsulfonyl, alkylsulfonyloxy, halogenalklsulfonyl halogenalkylsulfonyloxy, alkoxycarbonyl, acetyl, alkylcarbonyl, alkenylcarbonyl, pentafluorosulfanyl, amino, mono- and dialkylamino, cycloalkylamino, alkenyl, haloalkenyl, alkynyl, haloalkynyl, cyano, or nitro, or represent independently from each other aryl, aryloxy or heteroaryl which are optionally substituted with one or more substituents selected from halogen, alkyl, haloalkyl, alkoxy, haloalkoxy, alkoxycarbonyl, nitro, and cyano;
R⁶ and R⁷ independently from each other represent hydrogen, alkyl, haloalkyl, cycloalkyl, alkoxyalkyl, alkylmercaptoalkyl, alkenyl, or alkynyl, or represent independently from each other aryl, heteroaryl or heterocyclyl, optionally substituted with one or more substituents selected from halogen, alkyl, alkoxy, nitro, and cyano;
R⁸ represents -C(Z)R¹⁰, -C( Z)OR¹⁰, or -C(Z)NR¹¹R¹²;
Z represents O or S;
R⁹ represents hydrogen, alkyl, or haloalkyl;
R¹⁰ represents C₁-C₂ alkyl, substituted with one or more substituents selected from halogen, alkoxy, alkylmercapto and cyano, or represents C₃-C₆ alkyl, cycloalkyl, or benzyl, optionally substituted with one or more substituents selected from halogen, and alkoxy;
R¹¹ represents C₁-C₂ alkyl, substituted with one or more substituents selected from halogen, alkoxy, alkylmercapto, alkoxycarbonyl, alkylsulfinyl, alkylsulfonyl and cyano, or represents C₃-C₈ alkyl, cycloalkyl, cycloalkylalkyl, alkenyl, or alkynyl, optionally substituted with one or more substituents selected from halogen, haloalkyl, alkoxy, alkylmercapto, alkylsulfinyl, alkylsulfonyl, dialkylamino, alkoxycarbonyl, and dialkylaminocarbonyl, or represents arylalkyl, heterocyclylalkyl, aryl, heteroaryl, or heteroarylalkyl, optionally substituted with one or more substituents selected from halogen, alkyl, halogenalkyl, alkoxy, halogenalkoxy, alkoxycarbonyl, heterocyclyl, dialkylaminocarbonyl, cyano, nitro, dialkylamino, S(O)ₙ-alkyl, and S(O)ₙ-halogenalkyl;
R¹² represents hydrogen or alkyl, cycloalkyl, alkenyl, alkynyl, optionally substituted with one or more substituents selected from halogen, alkoxy, alkylmercapto, cyano, alkylsulfinyl, alkylsulfonyl, dialkylamino, alkoxycarbonyl, and dialkylaminocarbonyl, or represents arylalkyl, heterocyclylalkyl, aryl, or heteroaryl, optionally substituted with one or more substituents selected from halogen, alkyl, halogenalkyl, alkoxy, halogenalkoxy, alkoxycarbonyl, dialkylaminocarbonyl, cyano, nitro, dialkylamino, S(O)ₙ-alkyl, and S(O)ₙ-halogenalkyl;
n is 0, 1 or 2

2. Compounds of the formula (I) according to claim 1, in which
R¹, R², R³, R⁴, and R⁵ independently from each other represent hydrogen, halogen, alkyl, alkoxy, haloalkyl, alkoxyalkyl, cycloalkyl, haloalkoxy, alkylthio, haloalkylthio, alkylsulfonyl, alkylsulfonyloxy, halogenalkylsulfonyl, alkoxycarbonyl, acetyl, alkylcarbonyl, alkenylcarbonyl, dialkylamino, cycloalkylamino, alkenyl, haloalkenyl, alkynyl, haloalkynyl, cyano, or nitro;
R⁶ and R⁷ independently from each other represent hydrogen, alkyl, haloalkyl, cycloalkyl, alkoxyalkyl, alkylmercaptoalkyl, alkenyl, or alkynyl, or independently from each other represent aryl, optionally substituted with one or more substituents selected from halogen, alkyl, alkoxy, nitro and cyano;
R⁸ represents -C(Z)NR¹¹R¹²;
Z represents O or S;
R⁹ represents hydrogen, or alkyl;
R¹¹ represents C₁-C₂ alkyl, substituted with one or more substituents selected from halogen, alkoxy, alkylmercapto, alkoxycarbonyl, alkylsulfinyl, alkylsulfonyl and cyano, or represents C₃-C₈ alkyl, cycloalkyl, cycloalkylalkyl, alkenyl, or alkynyl, optionally substituted with one or more substituents selected from halogen, haloalkyl, alkoxy, alkylmercapto, alkylsulfinyl, alkylsulfonyl, dialkylamino, alkoxycarbonyl, and dialkylaminocarbonyl, or represents arylalkyl, heterocyclylalkyl, aryl, beteroaryl, or heteroarylalkyl, optionally substituted with one or more substituents selected from halogen, alkyl, halogenalkyl, alkoxy, halogenalkoxy, alkoxycarbonyl, heterocyclyl, dialkylaminocarbonyl, cyano, nitro, dialkylamino, S(O)ₙ-alkyl, and S(O)ₙ-halogenalkyl;
R¹² represents hydrogen or alkyl, cycloalkyl, alkenyl, alkynyl, optionally substituted with one or more substituents selected from halogen, alkoxy, alkylmercapto, cyano, alkylsulfinyl, alkylsulfonyl, dialkylamino, alkoxycarbonyl, and dialkylaminocarbonyl, or represents arylalkyl, heterocyclylalkyl, aryl, or heteroaryl, optionally substituted with one or more substituents selected from halogen, alkyl, halogenalkyl, alkoxy, halogenalkoxy, alkoxycarbonyl, dialkylaminocarbonyl, cyano, nitro, dialkylamino, S(O)ₙ-alkyl, and S(O)ₙ-halogenalkyl;
n is 0, 1 or 2

3. Compounds of the formula (I) according to claim 1 or 2, in which
R¹, R², R³, R⁴, and R⁵ independently from each other represent hydrogen, halogen, alkyl, alkoxy, or haloalkyl;
R⁶ and R⁷ independently from each other represent hydrogen, alkyl, or haloalkyl;
R⁸ represents -C(S)NR¹¹R¹²;
R⁹ represents hydrogen;
R¹¹ represents C₁-C₂ alkyl, substituted with one or more substituents selected from halogen, alkoxy, alkylmercapto, alkoxycarbonyl, alkylsulfinyl, alkylsulfonyl, or cyano, or represents C₃-C₈ alkyl, cycloalkyl, cycloalkylalkyl, or alkenyl, optionally substituted with one or more substituents selected from halogen, haloalkyl, alkoxy, alkylmercapto, alkylsulfinyl, alkylsulfonyl, and alkoxycarbonyl, or represents arylalkyl, heterocyclylalkyl, aryl, heteroaryl, or heteroarylalkyl, optionally substituted with one or more substituents selected from halogen, alkyl, halogenalkyl, alkoxy, halogenalkoxy, alkoxycarbonyl,and heterocyclyl; and
R¹² represents hydrogen.

4. Compounds of tha formula (I) according to claim 3, in which
R⁶ represents alkyl,
R⁷ represents hydrogen, and
R¹, R², R³, R⁴, R⁵, R⁸, R⁹, R¹¹ and R¹² are as defined in claim 3.

5. Compounds of formula (Tb) 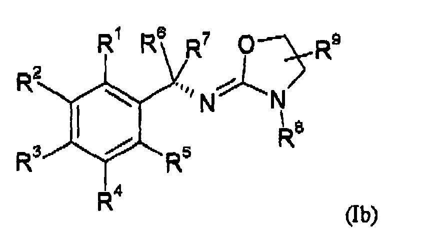 in which R¹ to R¹² are as defined in any of claims 1 to 4,

6. Method for the preparation of compounds of the formula (1) or (Ib) according to any of claims 1 to 5 in which R⁸ is -C(S)NR¹¹R¹², and R¹² is hydrogen, wherein
a compound of the formula (II) or (IIb) 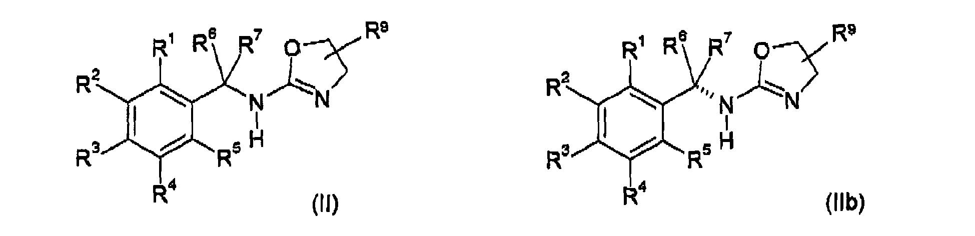 in which
R¹, R², R³, R⁴, R⁴, R⁷ and R⁹ are as defined above
is reacted with thiocarbonyldiimidazole 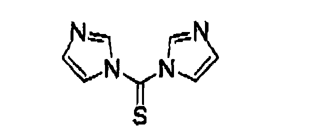 and a compound of the formula (IV)
R¹²-NH₂ (IV)
in which
R¹¹ is as defined above,
in an aprotic solvent.

7. Composition comprising at least one compound of the formula (1) or (Ib) according to any of claims 1 to 5 and customary extenders and/or surfactants.

8. Method for controlling pests, wherein a compound of the formula (I) or (Ib) according to any of claims 1 to 5 or a composition according to claim 7 is allowed to act on the pests and/or their habitat.

9. Use of compounds of the formula (I) or (Ib) according to any of claims 1 to 5 or of compositions according to claim 7 for controlling pests.
